# EUROPEAN PATENT SPECIFICATION

(11) **EP 4 683 599 B1**
(45) Date of publication and mention of the grant of the patent: **11.11.2026**
(21) Application number: 24719684.3
(22) Date of filing: 12.03.2024
(51) Int. Cl.: A61F 13/551

(54) **ABSORBENT ARTICLE PACKAGED IN A MATERIAL COMPRISING NATURAL FIBERS**
SAUGFÄHIGER ARTIKEL VERPACKT IN EINEM MATERIAL MIT NATURFASERN
ARTICLE ABSORBANT EMBALLÉ DANS UN MATÉRIAU COMPRENANT DES FIBRES NATURELLES

(30) Priority: 21.03.2023 US 202363453569 P
(43) Date of publication of application: 28.01.2026
(73) Proprietor: The Procter & Gamble Company, Cincinnati, OH 45202 (US)
(72) Inventor: MALDONADO, Clarissa, Cincinnati, Ohio 45202 (US); MACURA, Anna, Elizabeth, Cincinnati, Ohio 45202 (US); DENG, Rong, Cincinnati, Ohio 45202 (US)
(74) Representative: P&G Patent Germany
(86) International application number: PCT/US2024/019481
(87) International publication number: WO 2024/196633

(56) References cited:
- CN-A- 115 103 806
- JP-A- 2021 153 917
- US-A- 5 295 988
- US-A1- 2004 232 024
- US-A1- 2023 036 459

## Description

### FIELD OF THE INVENTION

This invention relates to an individually packaged absorbent article provided in a folded configuration and comprising a wrapper releasably affixed to the absorbent article, where the wrapper comprises a material comprising natural fibers.

### BACKGROUND OF THE INVENTION

Users/consumers of absorbent articles, such as feminine hygiene pads, have developed a number of varying expectations and preferences for such products over the years, as the products themselves have evolved. These expectations and preferences include (in no particular order) (1) that the absorbent article have suitable absorption performance such that it will readily accept, absorb, contain, isolate, and effectively retain all fluid discharged, away from the user's skin and without leaking, over a normal time of use/wear; (2) that the absorbent article be as thin (non-bulky), flexible and pliable as possible for purposes of comfort, accommodation of the wearer's body movements, and discreetness of wear under clothing; and (3) that the absorbent article and its wrapping provide for convenient and discreet carrying, easy opening and access to the absorbent article, as well as convenient disposal of used absorbent articles. More recently, consumers of absorbent articles, such as feminine hygiene pads, are also seeking products that comprise natural materials, bio-sourced materials, and/or recycled materials. And, in the context of disposal, consumers are seeking products comprising components or packaging that is bio-degradable, compostable, recyclable, reusable, and/or otherwise contributes to reduced landfill waste.

Modern absorbent articles, such as feminine hygiene pads, are highly optimized to meet consumers' varying expectations and preferences. For example, many feminine hygiene pads are folded and compacted so that, once packaged, the articles are thin and discrete to carry in a purse. Such feminine hygiene pads are typically folded and wrapped individually with a thin, flexible wrapper, which may subsequently be used for disposal of used product. The wrappers are typically made of plastic, such as polyethylene film. Plastic is generally preferred because it can withstand the rigors of the manufacturing process, due to its ability to flex and stretch. However, there is growing public demand for alternatives to plastic and non-plastic based wrappers. The replacement of a plastic wrapper material with a wrapper material comprising natural fibers, such as paper, in existing manufacturing processes may present challenges; materials comprising natural fibers may have less resistance to the stresses normally encountered during such manufacturing processes.

CN115103806A discloses a two-part packaging system for products like diapers, comprising a primary package for the article and a secondary package (e.g., an inflatable air column bag) that provides compression resistance and a moisture barrier for transport and storage.

US5295988A describes an individually wrapped absorbent article where a tensioned elastic element is held flat by the wrapper. Upon unwrapping, the tension is released, causing the article to form a cupped shape for conformity to the wearer's body.

There is therefore a need to provide a disposable absorbent article individually wrapped in a material comprising natural fibers, which can be manufactured under existing process conditions at high speed.

### SUMMARY OF THE INVENTION

The invention provides an individually packaged absorbent article according to the claims.

### BRIEF DESCRIPTION OF THE DRAWINGS

Figure 1 is a perspective view of a feminine hygiene product, in an unopened state, having a folded feminine hygiene pad wrapped in a wrapper;
Figure 2 is a top plan view of a feminine hygiene product, in an opened state, having an unfolded feminine hygiene pad on an unfolded wrapper;
Figure 3 is a cross-sectional view of the feminine hygiene pad and wrapper of Figure 2 in a folded configuration, taken along the longitudinal center line (L);
Figure 3A is top view of a wrapper indicating fold lines;
Figure 3B is a schematic side view of a feminine hygiene pad folded in four sections in a roll fold configuration, with a main fold nose oriented toward the top of the figure.
Figure 3C is a schematic side view of a feminine hygiene pad folded in four section in a book jacket fold configuration, with a main fold nose oriented toward the top of the figure. Figure 4 is a side view of machinery for producing crepe paper.
Figure 5 is a close-up view of a portion of the machinery of Figure 4, including illustrations of several stages in the production of the crepe paper.
Figure 6 is a top plan view of a feminine hygiene product, in an opened state, having an unfolded feminine hygiene pad on an unfolded wrapper, showing where a release agent and sealing aid are disposed on the wearer-facing surface of the wrapper.
Figure 7 is a top plan view of a feminine hygiene product, in an opened state, having an unfolded feminine hygiene pad on an unfolded wrapper, showing where a release agent and sealing aid are disposed on the wearer-facing surface of the wrapper.
Figure 8A is a cross sectional view of a wrapper and the one or more layers of coatings applied to the wrapper.
Figure 8B is a cross sectional view of a wrapper and the one or more layers of coatings applied to the wrapper.
Figure 8C is a cross sectional view of a wrapper and the one or more layers of coatings applied to the wrapper.
Figure 8D is a cross sectional view of a wrapper and the one or more layers of coatings applied to the wrapper.
Figure 9A is a top plan view of a feminine hygiene product, in an opened state, having an unfolded feminine hygiene pad on an unfolded wrapper, showing where a sealing aid is disposed on the wrapper.
Figure 9B is a top plan view of a feminine hygiene product, in an opened state, having an unfolded feminine hygiene pad on an unfolded wrapper, showing where a sealing aid is disposed on the wrapper.
Figure 9C is a top plan view of a feminine hygiene product, in an opened state, having an unfolded feminine hygiene pad on an unfolded wrapper, showing where a sealing aid is disposed on the wrapper.
Figure 9D is a top plan view of a feminine hygiene product, in an opened state, having an unfolded feminine hygiene pad on an unfolded wrapper, showing where a sealing aid is disposed on the wrapper.
Figure 10 is a graph of web modulus (at 1-2% strain) versus Stretch (%) for eight different wrapping materials.
Figure 11 is a graph showing the influence of paper properties on folding angle for three paper wrapping materials shown in Figure 10.
Figure 12A-12C show equipment used in the Ultra Sensitive 3 Point Bending Method.

### DETAILED DESCRIPTION

### Definitions

The term "absorbent article" as used herein refers to devices which absorb and contain exudates, and, more specifically, refers to devices which are placed against or in proximity to the body of the wearer to absorb and contain the various exudates discharged from the body. Absorbent articles of the present disclosure include, but are not limited to, diapers, adult incontinence briefs, training pants, diaper holders, diaper outer covers, absorbent inserts for the diaper outer covers, feminine hygiene pads/menstrual pads, incontinence pads, liners, pantiliners, tampons, durable feminine hygiene pants/menstrual pants, disposable swim pants, and the like.

The term "renewable" is synonymous with the terms "biobased," "sustainable," "sustainably derived," or "from sustainable sources" and means bio-derived (derived from a renewable resource, e.g., plants) or "non-geologically derived." "Geologically derived" means derived from, for example, petrochemicals, natural gas, or coal. "Geologically derived" materials cannot be easily replenished or regrown (e.g., in contrast to plant- or algae-produced oils).

As used herein, the term "renewable component" refers to a component that is derived from renewable feedstock and contains renewable carbon. A renewable feedstock is a feedstock that is derived from a renewable resource, e.g., plants, and non-geologically derived. A material may be partially renewable (less than 100% renewable carbon content, from about 1% to about 50% renewable carbon content) or 100% renewable (100% renewable carbon content). A renewable material may be blended with a nonrenewable material.

"Renewable carbon" may be assessed according to the "Assessment of the Biobased Content of Materials" method, ASTM D6866.

The term "substantially free of" or "substantially free from" as used herein refers to either the complete absence of an ingredient or a minimal amount thereof merely as impurity or unintended byproduct of another ingredient. A composition that is "substantially free" of/from a component means that the composition comprises less than about 5%, 3%, 2%, 1%, 0.5%, 0.25%, 0.1%, 0.05%, or 0.01%, or even 0%, by weight of the composition, of the component.

The term "natural fibers" as used herein, refers to fibers which comprise cellulose-based fibers, bamboo based fibers, and the like. Natural fibers also refers to nonwoody fibers, such as cotton, abaca, kenaf, sabai grass, flax, esparto grass, straw, jute hemp, bagasse, milkweed floss fibers, and pineapple leaf fibers; and woody, wood, or pulp fibers such as those obtained from deciduous and coniferous trees, including softwood fibers, such as northern and southern softwood kraft fibers; hardwood fibers, such as eucalyptus, maple, birch, and aspen. Pulp fibers can be prepared in high-yield or low-yield forms and can be pulped in any known method of chemical or mechanical pulping, including kraft, sulfite, high-yield pulping methods. The natural fibers of the present disclosure may be recycled natural fibers, virgin natural fibers or mixes thereof. Additionally, for good mechanical properties in natural fibers, it can be desirable that the natural fibers be relatively undamaged and largely unrefined or only lightly refined. A natural fiber pulp may be characterized by a Canadian Standard Freeness value, which characterizes the change in the drainage rate of a pulp and is widely used to represent the change in fiber properties during beating and refining. The Canadian Standard Freeness value has been shown to relate to the surface conditions and swelling of the pulp fibers and may be used to control fiber properties by selecting the optimal level of refining energy needed for a selected grade of pulp. The Canadian Standard Freeness value may vary depending on the fiber type, fiber morphology, and the concentration of mass in the fiber suspension. The Canadian Standard Freeness value may be determined according to standardized test procedures, e.g., TAPPI T-227 om-17 (2017).

The term "cellulose-based fibers," as used herein, includes cellulose fibers, such as wood fiber, and cotton, regenerated cellulose fiber, such as rayon or cuprammonium rayon, and high pulping yield fibers. The term "cellulose-based fibers" also includes chemically treated natural fibers, such as mercerized pulps, chemically stiffened or crosslinked fibers, or sulfonated fibers. Also included are mercerized natural fibers, regenerated natural cellulosic fibers, cellulose produced by microbes, the rayon process, cellulose dissolution and coagulation spinning processes, and other cellulosic material or cellulosic derivatives. Other cellulose-based fibers included are paper broke or recycled fibers and high yield fibers. High yield pulp fibers are those fibers produced by pulping processes providing a yield of about 65% or greater, more specifically about 75% or greater, and still more specifically about 75% to about 95%, where yield is the resulting amount of processed fibers expressed as a percentage of the initial wood mass. Such pulping processes include bleached chemithermomechanical pulp (BCTMP), chemithermomechanical pulp (CTMP), pressure/pressure thermomechanical pulp (PTMP), thermomechanical pulp (TMP), thermomechanical chemical pulp (TMCP), high yield sulfite pulps, and high yield Kraft pulps, all of which leave the resulting fibers with high levels of lignin but are still considered to be natural fibers. High yield fibers are well known for their stiffness in both dry and wet states relative to typical chemically pulped fibers.

The term "machine direction" or "MD", as used herein, refers to the path that material, such as a web, follows through a manufacturing process.

The term "cross-machine direction" or "CD", as used herein, refers to the path that is perpendicular to the machine direction in the plane of the web.

With respect to an absorbent article, such as a feminine hygiene pad, that is open and laid out flat on a horizontal planar surface, "lateral" refers to a direction perpendicular to the longitudinal direction and parallel the horizontal planar surface. "Width" refers to a dimension measured along a lateral direction.

With respect to an absorbent article, such as a feminine hygiene pad, that is open and laid out flat on a horizontal planar surface and having a length measured from its forwardmost end to its rearwardmost end, "longitudinal" refers to a direction parallel with the line along which the length is measured, and parallel to the horizontal planar surface. "Length" refers to a dimension measured in the longitudinal direction.

With respect to an absorbent article, such as a feminine hygiene pad, the terms "front", "rear", "forward" and "rearward" relate to features or regions of the article in a position as it would ordinarily be worn by a user, and the front and rear of the user's body when standing.

With respect to an absorbent article, such as a feminine hygiene pad, that is open and laid out flat on a horizontal planar surface, "z-direction" refers to a direction perpendicular to the horizontal planar surface. When the article is being worn by a user (and thus in a curved configuration), "z-direction" at any particular point location on the article refers to a direction normal to the wearer-facing surface of the article at the particular point location.

With respect to an absorbent article, such as a feminine hygiene pad, "wearer-facing" is a relative locational term referring to a feature of a component or structure of the article that, when in use, lies closer to the wearer than another feature of the component or structure that lies along the same z-direction. For example, a topsheet has a wearer-facing surface that lies closer to the wearer than the opposite, outward-facing surface of the topsheet.

With respect to an absorbent article, such as a feminine hygiene pad, "outward-facing" or "garment-facing" is a relative locational term referring to a feature of a component or structure of the article that, when in use, lies farther from the wearer than another feature of the component or structure that lies along the same z-direction. For example, a topsheet has an outward-facing or garment-facing surface that lies farther from the wearer than the opposite, wearer-facing surface of the topsheet.

### Description

### Absorbent Feminine Hygiene Pad

An absorbent feminine hygiene pad may have any shape known in the art for feminine hygiene articles, including the generally symmetric "hourglass" shape, as well as pear shapes, bicycle-seat shapes, trapezoidal shapes, wedge shapes or other shapes that have one end wider than the other. Sanitary napkins and pantiliners can also be provided with lateral extensions known in the art as "flaps" or "wings"). Such extensions can serve a number of purposes, including, but not limited to, protecting the wearer's panties from soiling and keeping the sanitary napkin secured in place. The absorbent article has a wearer-facing side that contacts the user's body during use and an opposite, garment-facing or outward-facing side that contacts the user's undergarments during use.

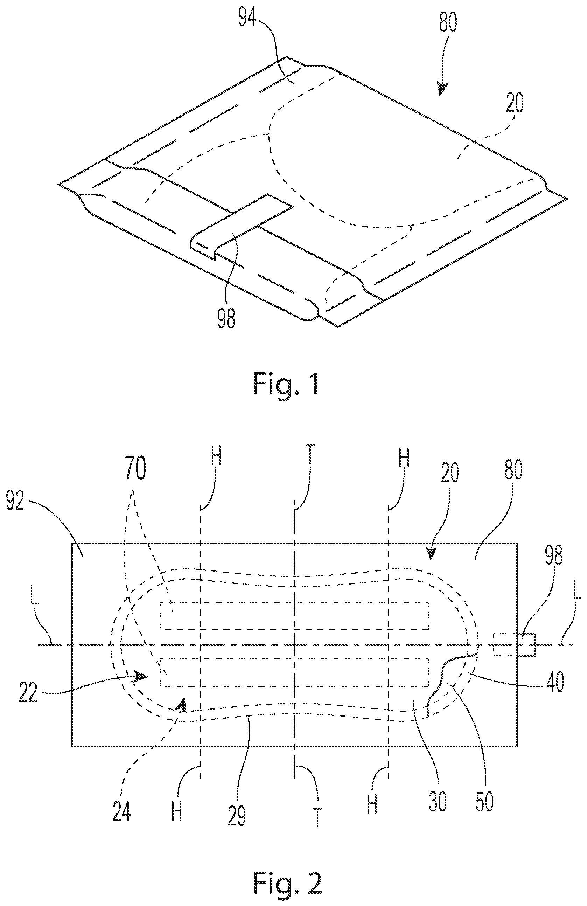

Figure 1 is a perspective view of a disposable feminine hygiene product and Figure 2 is a top plan view of a disposable feminine hygiene product according to the present disclosure, in an opened state, having an unfolded feminine hygiene pad 20 on an unfolded wrapper 80. The pad 20 (and its component layers) has a wearer-facing surface 22, and a garment-facing surface 24 opposite to the wearer-facing surface 22. The feminine hygiene pad 20 shown in Figure 2 is viewed from the wearer-facing surface 22. The feminine hygiene pad 20 has a perimeter 29 which defines the outermost edge of the pad 20. The feminine hygiene pad 20 has two centerlines, a longitudinal centerline L and a lateral centerline T.

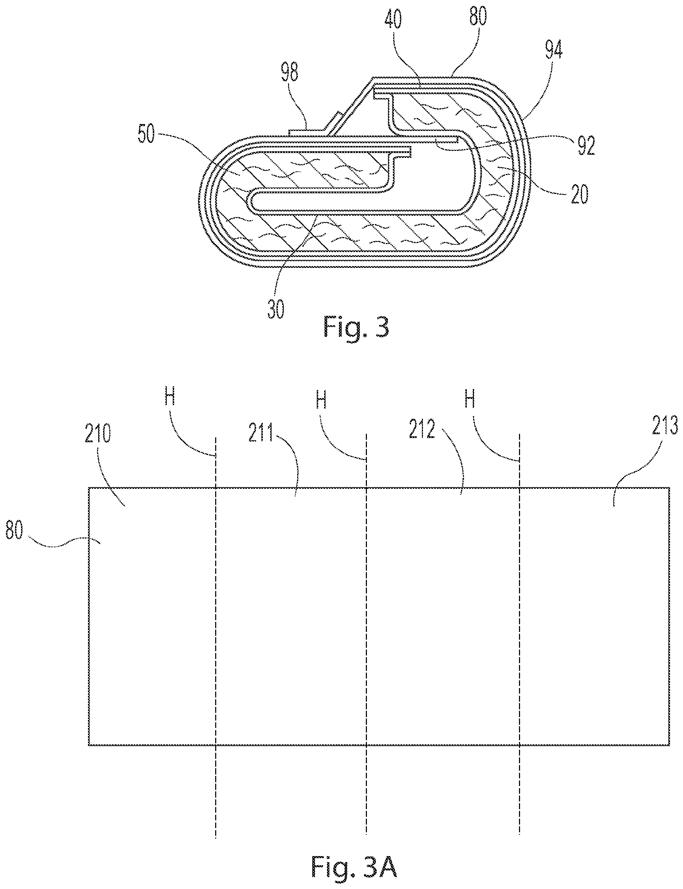

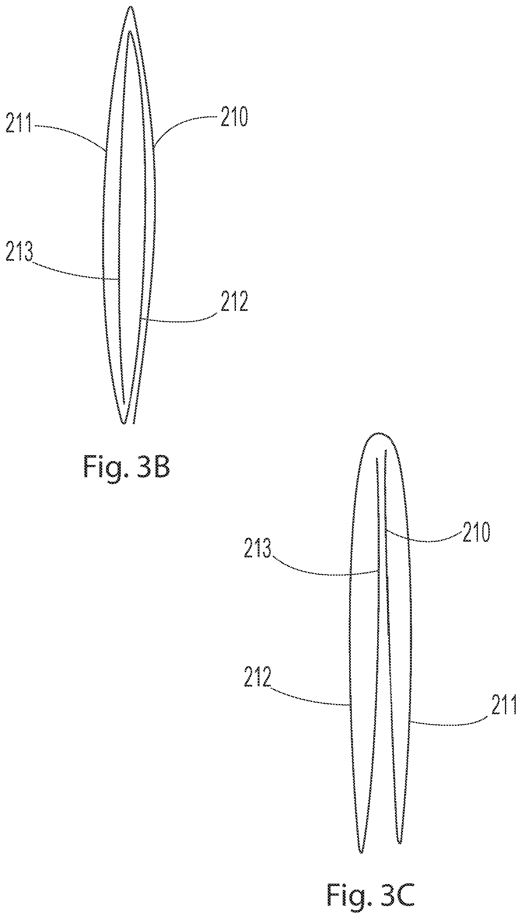

Figure 3 is a cross-sectional view of the feminine hygiene pad and wrapper of Figure 2 in a folded configuration, taken along the longitudinal center line (L). The pad 20 may be folded together with the wrapper sheet 80 along the two folding lines H shown in Figure 2. The number of folding lines may vary depending on the size or the design of the pad 20. For example, the number of the folding lines can be one, two, three, or more, if desired. After being folded, the outer surface 94 (the surface facing away from the feminine hygiene pad) of one end of the wrapper 80 is joined to the outer surface 94 of the other end of the wrapper 80 by a resealable tape 98, thereby forming the individual package shown in Figure 3. A soiled pad may be wrapped in the wrapper 80 and the resealable tape 98 may be used to reseal the wrapper 80 in order to dispose of the soiled pad. Alternatively, the feminine hygiene pad 20 may be placed into a pouch formed by the wrapper, in a folded or unfolded configuration. In some embodiments, the wrapper 80 may be sealed without a reseal tape.

In some embodiments, the wrapper may be folded along three fold lines H, such as illustrated in Figure 3A. The wrapper in Figures 3A-3C are illustrated without an absorbent article for ease of illustration. However, it is to be appreciated that the absorbent article would be folded with the wrapper. A wrapper folded along three fold lines H may be folded in various configurations such as a roll fold configuration and a book jacket fold configuration. The roll fold configuration is illustrated in Figure 3B. In a roll fold configuration, rear section 213 is folded along about rear lateral fold line H over rear-mid section 212; then both sections 213 and 212 together are folded about middle lateral fold line H over front-mid section 211; and lastly, the three section 213, 212 and 211 together are folded about front lateral fold line H over front section 210. The foregoing procedure results in a front-section-out roll fold configuration, in which front section 210 is disposed on the outside of the folded pad configuration as shown in Figure 3B. It will be appreciated that an alternative roll fold configuration as described above may be formed by first folding the front section 210 over about front lateral fold line H, and preceding to fold rearward, resulting in a rear-section-out roll fold configuration, in which rear section 210 is disposed on the outside of the folded configuration. A front-section-out roll fold configuration as shown in Figure 3B may be desired, however, for purposes of user convenience, because it provides for front-to-rear unfolding that may be more intuitive for the user. It will be appreciated that a front-section-out roll fold configuration, or a rear-section-out roll fold configuration (if desired), may be created via steps that differ from those described above.

The second configuration is the book jacket fold configuration and is illustrated in Figure 3C. In a book jacket fold configuration, front section 210 is folded about front lateral fold line H over front-mid section 211; and rear second 212 is folded about rear lateral fold line H over rear-mid section 212 (these two folds can occur simultaneously or one after another in either order). Finally, the two remaining portions comprising front section 210 folded over front-mid section 211 as one portion, and rear section 213 folded over rear-mid section 212, are folded about middle lateral fold line H to bring them together to create the book jacket fold configuration as illustrated in Figure 3C. It is to be appreciated that there are other folded configurations known in the art.

Referring to FIGS. 2 and 3, an absorbent pad 20 may include a liquid permeable topsheet 30, a liquid impermeable backsheet 40 and an absorbent layer/core 50 disposed between the topsheet and the backsheet. The topsheet and the backsheet may be bonded together, at the perimeter edge of the absorbent pad, by any suitable mechanism, including but not limited to adhesive bonding, thermal/heat bonding, pressure bonding, ultrasonic bonding, dynamic mechanical bonding, a crimp seal, or by any other suitable bonding method, thereby retaining and holding the absorbent layer/core 50 in place between the topsheet 30 and the backsheet 40.

The absorbent pad 20 may include opposing wings (not shown) extending laterally from the longitudinal side edges of the pad by a comparatively greater width dimension than the main portion of the pad. Wings may be formed of lateral extensions of the material forming the topsheet 20, backsheet 30, or both together. The wings of the absorbent pads of the present disclosure may be integrally formed as part of the topsheet. In some forms, the wings may be integrally formed as part of the backsheet. In some forms, the wings may be integrally formed as part of the topsheet and the backsheet. In some forms, the wings may be integrally formed with additional layers - described herein - of the absorbent article. Yet in other forms, the wings may be formed discretely and joined to the chassis. The outward-facing surface of the backsheet forming the undersides of the main portion and the outward-facing surface of the wings may have deposits of adhesive 70 thereon. If present, the wings may be folded over the wearer-facing surface of the pad and, following such folding, a release film or paper may be applied to the portions of the wings having adhesive deposited thereon, to cover and protect the adhesive prior to use.

Adhesive deposits 70 may be provided to enable the user to adhere the pad to the inside of her underpants in the crotch region thereof. Adhesive deposits 70 on the outward-facing surface of the wings allow the user to secure the wings around the edges of the leg openings of the underpants and adhere them to the outside/underside of the underpants in the crotch region, providing supplemental holding support an/d helping guard the leg opening edges of the underpants against soiling. When pad 20 is wrapped and folded, adhesive deposits 70 may be covered by the wrapper 80, to shield them from contact with other surfaces until the user is ready to remove the wrapper 80 and place the pad 20 for use. Adhesive deposits 70 on the wings and/or the pad 20 may be covered by one or more removable sheets of release film or paper.

The topsheet 30 may be formed of any suitable liquid permeable web material. Referring back to FIGS. 2 and 3, the topsheet 30 is positioned adjacent a wearer-facing surface of the absorbent layer 50 and may be joined thereto and/or to the backsheet 40 by any suitable attachment or bonding method. The topsheet 30 and the backsheet 40 may be joined directly to each other at the perimeter edge of the absorbent pad (outside of the absorbent layer 50) and/or may be indirectly joined by directly joining them respectively to wearer-facing and outward-facing surfaces of the absorbent layer 50 or additional optional layers included with the pad.

Topsheet 30 may be formed of any liquid pervious web material that is suitably compliant, soft feeling, and non-irritating to the wearer's skin. Suitable topsheet materials include a liquid pervious material that contacts the body of the wearer and permits menstrual fluid discharges to rapidly penetrate through it. Some suitable examples of topsheet materials include films, nonwovens, laminate structures including film / nonwoven layers, film / film layers, and nonwoven / nonwoven layers. Other exemplary topsheet materials and designs are disclosed in U.S. Patent Application Publication Nos. 2016/0129661, 2016/0167334, and 2016/0278986.

A suitable topsheet can be made of various materials such as woven and nonwoven materials; apertured film materials including apertured formed thermoplastic films, apertured plastic films, and fiber-entangled apertured films; hydro-formed thermoplastic films; porous foams; reticulated foams; reticulated thermoplastic films; thermoplastic scrims; or combinations thereof. Some suitable examples of films that can be utilized as topsheets are described in US 3,929,135; US 4,324,246; US 4,342,314; US 4,463,045; US 5,006,394; US 4,609,518; and US 4,629,643.

Nonlimiting examples of woven and nonwoven web materials that may be suitable for use as the topsheet include fibrous materials made from natural fibers, modified natural fibers, synthetic fibers, or combinations thereof. Some suitable examples are described in U.S. Patent Nos. 4,950,264, 4,988,344; 4,988,345; 3,978,185; 7,785,690; 7,838,099; 5,792,404; and 5,665,452.

In some examples, the topsheet may comprise tufts as described in US 8,728,049; US 7,553,532; US 7,172,801; US 8,440,286; US 7,648,752; and US 7,410,683. The topsheet may have a pattern of discrete hair-like fibrils as described in US 7,655,176 or US 7,402,723. Additional examples of suitable topsheet materials include those described in US 8,614,365; US 8,704,036; US 6,025,535 and US 2015/041640. Another suitable topsheet may be formed from a three-dimensional substrate as detailed in US 2017/0258647. The topsheet may have one or more layers, as described in US 2016/0167334; US 2016/0166443; and US 2017/0258651. The topsheet may be apertured, as described in US 5,628,097.

As contemplated herein, component nonwoven web material from which topsheet 30 be cut may be a nonwoven web material that includes or consists predominately (by weight) or entirely of cellulosic plant fibers such as fibers of cotton, flax, hemp, jute or mixtures thereof, that are either naturally hydrophilic or suitably processed so as be rendered hydrophilic (or have increased hydrophilicity) and processed to be suitably soft. Plant-based fibers may be preferred to appeal to consumer preferences for natural products. In other examples, semisynthetic fibers derived from cellulosic material, such as rayon (including viscose, lyocell, MODAL (a product of Lenzing AG, Lenzing, Austria) and cuprammonium rayon) may be used. In some examples a topsheet cut from a carded nonwoven including or consisting predominately (by weight) or entirely of cotton fibers may be preferred. In some examples, the nonwoven web material may be formed via a carding process. In some other examples the nonwoven web material may be formed in a co-forming process in which plant-based fibers of finite lengths are physically blended or mixed with streams of filaments of indefinite lengths, spun from polymeric resin, and laid down on a forming belt to form a web as described in, for example, US 8,017,534; US 4,100,324; US 2003/0200991; US 5,508,102; US 2003/0211802; EP 0 333 228; WO 2009/10938; US 2017/0000695; US 2017/0002486; US 9,944,047; 2017/0022643 and US 2018/0002848.

For purposes of limiting bulk and caliper (thickness) of the pad, it may be desired that the topsheet be disposed in direct face-to-face relationship with the absorbent layer, with no intervening layer disposed therebetween. Alternatively, the pad may comprise an intervening layer, such as a secondary topsheet and/or an acquisition layer, positioned between the topsheet and the absorbent core. The secondary topsheet and/or an acquisition layer may be formed of a nonwoven web material, such as a spunlace nonwoven. Suitable spunlace nonwovens are discussed in additional detail in U.S. Patent Publication No. 2015/0351976. In some forms, the secondary topsheet may comprise superabsorbent similar to the superabsorbent in the absorbent core or different than the absorbent core. The secondary topsheet may comprise a first end and an opposing second end and a pair of longitudinally opposing side edges and connecting the first end and the second end. The secondary topsheet may be asymmetrical or symmetrical about the longitudinal centerline.

Any suitable absorbent layer/core known in the art may be utilized. The absorbent layer/core may be any absorbent member which is generally compressible, conformable, non-irritating to the wearer's skin, and capable of absorbing and retaining liquids such as urine, menses, and/or other body exudates. The absorbent layer/core may be manufactured from a wide variety of liquid-absorbent materials commonly used in disposable absorbent articles, such as comminuted wood pulp (generally referred to as airfelt). The absorbent layer/core may comprise superabsorbent polymers (SAP) and less than 15%, less than 10%, less than 5%, less than 3%, or less than 1% of airfelt, or be completely free of airfelt. Examples of other suitable absorbent materials comprise creped cellulose wadding, meltblown polymers including coform, chemically stiffened, modified or cross-linked cellulosic fibers, tissue including tissue wraps and tissue laminates, absorbent foams, absorbent sponges, superabsorbent polymers, absorbent gelling materials, or any equivalent material or combinations of materials.

The configuration and construction of the absorbent layer/core may vary (e.g., the absorbent core may have varying caliper zones, a hydrophilic gradient, a superabsorbent gradient, or lower average density and lower average basis weight acquisition zones; or may comprise one or more layers or structures). In some forms, the absorbent layer/core may comprise one or more channels, such as two, three, four, five, or six channels.

The absorbent layer/core of the present disclosure may comprise one or more adhesives, for example, to help immobilize the SAP or other absorbent materials within a core wrap and/or to ensure integrity of the core wrap, in particular when the core wrap is made of two or more substrates. The core wrap may extend to a larger area than required for containing the absorbent material(s) within.

Absorbent layers/cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen.

Other forms and more details regarding channels and pockets that are free of, or substantially free of absorbent materials, such as SAP, within absorbent cores are discussed in greater detail in U.S. Patent Application Publication Nos. 2014/0163500, 2014/0163506, and 2014/0163511, all published on June 12, 2014.

Other suitable materials for use in absorbent layers/cores comprise open celled foams or pieces thereof. The use of foams in absorbent cores is described in additional detail in U.S. Patent Nos. 6,410,820; 6,107,356; 6,204,298; 6,207,724; 6,444,716; 8,211,078, and 8,702,668.

In some forms, the absorbent layer/core structure may comprise a heterogeneous mass layer or may utilize methods or parameters such as those described in US patent application nos. 14/715,984, filed May 19, 2015; US Patent Application No. 14/750,399, June 25, 2015; US Patent Application No. 14/751,969 filed June 26, 2015; US Patent Application No. 15/078,132 filed March 23, 2016; US Patent Application No. 14/750,596 filed June 25, 2015; US Patent Application No. 15/084,902 filed March 30, 2016; US Patent Application No. 15/343,989 filed November 4, 2016; US Patent Application No. 15/344,273 filed November 4, 2016; US Patent Application No. 15/344,294 filed November 4, 2016; US Patent Application No. 14/704,110 filed May 5, 2015; US Patent Application No. 15/194,894 filed June 28, 2016; US Patent Application No. 15/344,050 filed November 4, 2016; US Patent Application No. 15/344,117 filed November 4, 2016; US Patent Application No. 15/344,177 filed November 4, 2016; US Patent Application No. 15/344,198 filed November 4, 2016; US Patent Application No. 15/344,221 filed November 4, 2016; US Patent Application No. 15/344,239 filed November 4, 2016; US Patent Application No. 15/344,255 filed November 4, 2016; US Patent Application No. 15/464,733 filed November 4, 2016; US Provisional Patent Application No. 62/437,208 filed December 21, 2016; US Provisional Patent Application No. 62/437,225 filed December 21, 2016; US Provisional Patent Application No. 62/437,241 filed December 21, 2016; or US Provisional Patent Application No. 62/437,259 filed December 21, 2016. The heterogeneous mass layer has a depth, a width, and a height.

In some forms, a combination of absorbent layer/core materials may be utilized. For example, forms are contemplated where a first layer of an absorbent core comprises a foam material or pieces thereof, as previously described, and a second layer of an absorbent core comprises an airlaid material. Such combinations are described in U.S. Patent Publication No. 2014/0336606 and U.S. Patent No. 9,649,228.

The backsheet 40 may be positioned adjacent an outward-facing surface of the absorbent layer/core 50 and may be joined thereto by any suitable attachment methods. For example, the backsheet 40 may be secured to the absorbent layer 50 by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment method may include heat bonds, pressure bonds, ultrasonic bonds, dynamic mechanical bonds, or any other suitable attachment mechanisms or combinations thereof. In other examples, it is contemplated that the absorbent layer 50 is not joined directly to the backsheet 40. The topsheet 30 may be joined to the backsheet 40 by the attachment methods described above. The topsheet and the backsheet may be joined directly to each other in the pad periphery and/or may be indirectly joined together by directly joining each to the absorbent core 50 by any suitable attachment method.

The backsheet 40 may be impervious, or substantially impervious, to liquids (e.g., urine, menstrual fluid) and may be manufactured from a thin plastic film, although other flexible liquid impervious materials may also be used. As used herein, the term "flexible" refers to materials which are compliant and will readily conform to the general shape and contours of the human body. The backsheet 40 may prevent, or at least substantially inhibit, fluids absorbed and contained within the absorbent layer 50 from escaping and reaching articles of the wearer's clothing which may contact the pad 20, such as underpants and outer clothing. However, in some instances, the backsheet 40 may be made and/or adapted to permit vapor to escape from the absorbent layer 50 (*i.e.,* the backsheet may be made to be breathable), while in other instances the backsheet 40 may be made so as not to permit vapors to escape (*i.e.,* it may be made to be non-breathable). Thus, the backsheet 40 may comprise a polymeric film such as thermoplastic films of polyethylene or polypropylene. A suitable material for the backsheet 40 is a thermoplastic film having a thickness of from about 0.012 mm (0.5 mil) to about 0.051 mm (2.0 mils), for example. Any suitable backsheet known in the art may be utilized with the present invention.

Some suitable examples of backsheets are described in US 5,885,265; US 4,342,314; and US 4,463,045. Suitable single layer breathable backsheets for use herein include those described for example in GB A 2184 389; GB A 2184 390; GB A 2184 391; US 4,591,523, US 3 989 867, US 3,156,242; WO 97/24097; US 6,623,464; US 6,664,439 and US 6,436,508.

The backsheet may have two layers: a first layer comprising a vapor permeable aperture-formed film layer and a second layer comprising a breathable microporous film layer, as described in US 6,462,251. Other suitable examples of dual or multi-layer breathable backsheets for use herein include those described in US 3,881,489, US 4,341,216, US 4,713,068, US 4,818,600; EP 203 821, EP 710 471; EP 710 472, and EP 0 793 952.

### Wrapper

The absorbent pads disclosed herein are individually packaged in wrappers. The wrappers comprise materials, preferably sheet materials, comprising natural fibers. Conventional wrappers are manufactured from a thin flexible material, which may be liquid impermeable. For example, conventional wrappers may be made from plastic films or nonwoven webs. The film or nonwoven web may be coated on an inner surface (surface facing the feminine hygiene pad) with a release agent, such as a silicone release agent, providing a releasably attached protective wrapper for the pad to protect the adhesive 70 prior to use, as shown in Figure 2. Such a protective wrapper also inhibits the adhesive 70 from inadvertently sticking to other portions of the pad 20 prior to use. Alternatively, some conventional, individually wrapped pads include a release sheet (not shown), which may be attached to the inside surface of an uncoated film or an uncoated nonwoven web. The release sheet may be formed by any suitable material known in the art, such as paper or plastic film. The release sheet may be coated on an inner surface (surface facing the feminine hygiene pad) with a release agent, such as a silicone release agent. Preferably, no extra steps are needed to remove the release sheet, as it is attached to the wrapper and removed from the pad at the same time as the wrapper. A coated wrapper may be preferred, as it eliminates the need for a release sheet. Importantly, though, for both of these conventional, individually wrapped pads, the wrapper itself and/or the coated release sheet are generally not recyclable (a paper release sheet that is coated with a silicone release agent is generally not recyclable). And, as noted above, there is growing public demand for materials that are recyclable or otherwise cause minimal landfill waste. Preferably, the wrappers according to the present disclosure are manufactured using materials that are recyclable and/or biodegradable.

The wrappers according to the present disclosure may comprise, consist essentially of, or consist of paper, where the term "paper" refers to a material manufactured in sheets from the pulp of wood or other fibrous substances and may comprise additives, such as synthetic fibers or biodegradable fibers. A wrapper comprising paper, such as Kraft paper, creped paper, and creped Kraft paper, may be recyclable, compostable, and/or biodegradable. In fact, existing recycling infrastructure may be better positioned to accept and process used creped paper than used plastic.

The paper may be Kraft paper, where "Kraft paper" refers to a paper produced from wood pulp by the Kraft process. The Kraft process removes almost all lignin from the wood, which results in almost pure cellulose fibers. The Kraft process is known in the art. A Kraft paper sheet is characterized by overall good strength properties and high porosity, and is thus suitable for a variety of applications.

The paper may comprise a blend of hardwood and softwood fibers, preferably a majority of softwood fibers, which may provide the toughness needed for surviving the creping process and for maintaining sufficient strength and bonding integrity within the fibers. The paper may comprise about 50% by weight to about 80% by weight of softwood fibers. The paper may comprise about 20% by weight to about 50% by weight of hardwood fibers. The hardwood fibers may be selected from the group consisting of maple, oak, elm, birch, poplar, aspen or eucalyptus fibers, and combinations thereof, preferably birch fibers. The softwood fibers may be selected from the group consisting of pines, firs, spruces, hemlocks, larch, and combinations thereof, preferably northern pine fibers. The paper may contain other natural fibers from plants such as cotton, flax, bamboo, wheat straw, red algae and/or other seaweeds, and hemp. The paper may also contain recycled fibers. Preferably, the presence of other natural fibers and/or recycled fibers do not impact the recyclability of the paper. The fibers used in the paper may have a Canadian Standard Freeness of at least 200mL, more specifically at least 300mL, more specifically still at least 400mL, and most specifically at least 500mL.

The paper has a MD web modulus in the 1%-2% strain range of less than 450N/cm to avoid variation of the placement of the wrapper on the pad and variation of the length of the fold in the pad. The paper has a stretch, calculated as MD stretch at break, of at least 9%, most preferably 15% or more.

The paper may have a basis weight of about 30 gsm to about 70 gsm, preferably from about 40 gsm to about 70 gsm, more preferably about 45gsm to about 65gsm, as determined via test method ISO 536 - Basis Weight as modified herein. Paper having lower basis weights, e.g., basis weights less than 30 gsm, may not be sufficiently opaque, based on consumer preferences.

The paper may have a water penetration time of at least 10 seconds, more preferably at least 17 seconds, according to test method EN 868-2:2017 EN ISO 1924-2. Because the absorbent products of the present disclosure may inadvertently come in contact with water or moisture, the wrappers of the present disclosure preferably exhibit some water repellency, to keep moisture and water from contaminating the absorbent pad inside the wrapper. Water repellency may be imparted or enhanced by the presence of lignin, a hydrophobic, high molecular weight natural polymer found in wood, wax, or a sizing agent in or on the paper. Other hydrophobic components may be formulated into a water-based varnish or a primer, which are applied to the surface of the paper. Preferably, any hydrophobic coating on the paper is applied in a minimal amount and does not impact the recyclability of the wrapper comprising the paper.

In some embodiments, a small amount of lignin may remain in the pulp, after the pulping process, to provide some hydrophobicity. To increase the hydrophobicity, lignin may also be coated onto paper using various methods.

In some embodiments, wax may be added and/or applied to the paper to increase the hydrophobicity. Waxes include emulsions of natural waxes, such as carnauba wax. beeswax, candelilla wax, rice bran wax, soybean wax, as well as petroleum-based waxes, such as paraffin wax, montanwax, and food grade synthetic waxes, such as oxidized LDPE and HDPE. Wax emulsions may be added to the pulp or a small coating of wax may be applied on the surface of the paper. The wax may be applied prior to subsequent processing, such as creping.

In some embodiments, sizing agents may be added and/or applied to the paper to increase the hydrophobicity. Sizing agents include internal sizing agents, which are added to the pulp during the production of the paper, and surface sizing agents, which are coated onto paper. Internal sizing agents include acidic type internal sizing agents, such as rosin and rosin derivatives, which contain aluminum resinate and are derived from natural resins and aluminum salts. Basic and neutral type internal sizing agents include synthetic sizing agents, such as alkylated ketene dimers (stable, hydrophobic waxy substances) and alkenyl succinic anhydrides (reactive, hydrophobic oily substances). Surface sizing agents include modified starches and gelatin, which can be naturally derived, as well as synthetic agents, such as styrenic polymers (e.g., styrene maleic anhydride, styrene acrylic emulsions, styrene acrylic acid, ethylene acrylinc acid, and polyurethane).

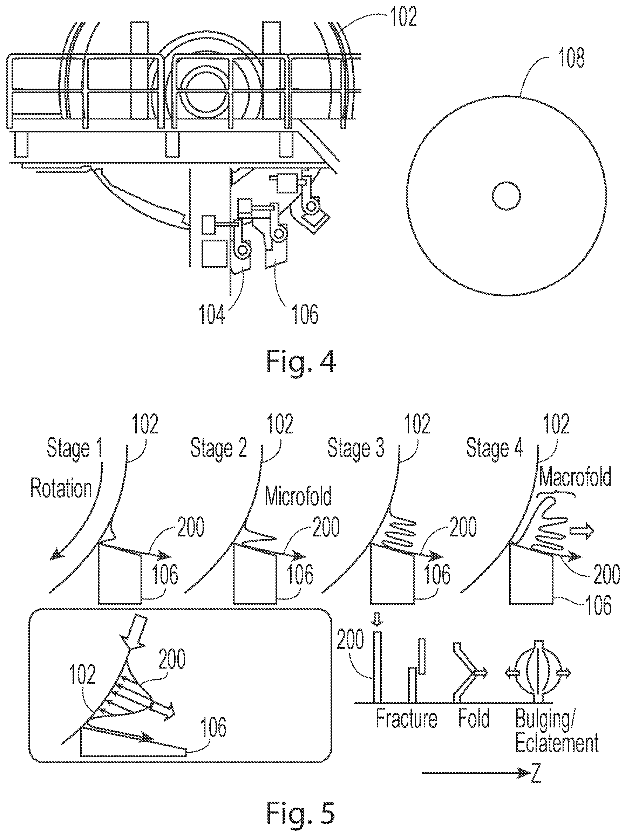

As previously discussed, paper wrappers are intended to be an alternative to traditional polymeric wrappers. However, for a paper wrapper to be a viable alternative, the paper needs to have properties that allow it to withstand the relatively rigorous manufacturing process and to adequately protect the absorbent article to be housed within the wrapper. One of the ways to enhance the properties of paper is to crepe the paper. "Creping" is a mechanical process for creating a low density, and increased caliper, paper ("crepe paper"). Figure 4 illustrates major components of machinery used to perform the creping process, as is well known in the art. Creping involves a rapidly rotating heated roller ("Yankee cylinder" or "Yankee dryer") 102 that dries paper advancing paper web, also referred to herein as a paper sheet, and provides a platform on which the creping occurs. The web is held fast onto a surface of the Yankee cylinder 102 by a mixture of naturally occurring pulp products, such as lignins and hemicellulose, and applied chemicals that form a thin, sticky layer ("Yankee coating") on the Yankee cylinder 102. The Yankee coating may be sprayed on the sheet or the Yankee cylinder by a coating sprayer 104. A fixed doctor blade ("crepe blade") 106 extends the width of the Yankee cylinder 102, removes the sheet from the Yankee cylinder 102 and forms the crepe paper, as described in more detail below. The formed crepe paper is then taken up on a parent roll 108.

Figure 5 is a close up view of a portion of the Yankee cylinder 102 proximate the crepe blade 106. Figure 5 shows four stages, during which folds form in the paper sheet 200. The fibers are attached to each other by hydrogen bonds. Impact of the paper sheet 200 against the crepe blade 106 generates large forces within the paper sheet 200, partially disrupting the inter-fiber hydrogen bonds, fracturing the Yankee coating-to-fiber interface, distorting the fibers, and forming micro- and/or macro-folds in the paper substrate. Consequently, the paper sheet expands in the z direction. Some fibers buckle and bend, as shown in Figure 5. Depending upon the adhesive strength, the expanded and buckled sheet releases from the Yankee cylinder 102 surface for a short distance, as shown in Stage 1. The greater the adhesive strength, the shorter this distance. Thus, a small fold of tissue or crepe forms, before the held sheet re-impacts against the crepe blade 106, and the process repeats.

Completed crepes are constantly moving away from the crepe pocket as the sheet 200 is wound up onto the parent roll 108. The crepe process shortens the length of the sheet 200 while increasing its caliper, thus the reel winding the parent roll 108 runs slower than the Yankee cylinder 102. The sheet caliper in expanded first by x-direction hydrogen bond disruption and secondly by the crepe action. However, the crepe itself is not necessarily uniform. The crepe may consist of large folds (macrofolds, as shown in Stage 4) interspersed with many smaller folds (microfolds, as shown in Stage 3).

The crepe bars in crepe paper make the crepe paper stretchable, at least in the longitudinal direction, and after being stretched, the crepe paper may remain somewhat elastic. Creped paper is generally more elastic than paper that has not been subjected to a creping process. Thus, stretchability and elasticity make crepe paper a suitable replacement for plastic, in the context of wrappers for absorbent pads. Preferably, the absorbent pad may be wrapped in the crepe paper wrapper on a high-speed production line, using essentially the same process and equipment as is used for current absorbent pads wrapped in plastic film. In other words, it would be desirable to provide such an article (e.g., pad) without compromising process conditions, such as, for example, the line speed.

### Process for Making Individually Wrapped Absorbent Articles

Processes for manufacturing individually packaged, folded feminine hygiene articles are known in the art and described in, for example, European Patent Application No. 1941852A1.

Generally, in existing high speed production lines, the material of the wrapper 80 is typically provided in a continuous web. The adhesive 70 disposed on the outward-facing surface of the backsheet 40 of the absorbent pad (as shown in Fig. 2) may be provided in selected areas directly onto the wrapper 80 material continuous web, and then absorbent pads may be provided onto each respective adhesive, by adhering thereon the outward-facing side of the backsheet 40 of each absorbent pad. The wrapper material continuous web may be folded together with the applied absorbent article or pad, and cut and sealed in order to form individually packaged articles. When the user, after unfolding the package and breaking the one or more seals, separates the absorbent article or pad from the wrapper material, at least some of the adhesive remains on the outward-facing surface of the backsheet, as the respective surface of the wrapper is generally coated with a release agent and has less adhesion to the adhesive material.

The wrapper material may be subjected to substantial stress in known high speed processes to produce individually packaged absorbent articles. The stress can be both mechanical and thermal. Thermal stresses may be due, at least in part, to the adhesive material that is typically a hot melt adhesive being disposed on the wrapper material in molten state, which is at relatively high temperature. Mechanical stresses may be due, at least in part, to the tension and forces placed on the wrapper during the manufacturing process. For many years, the absorbent feminine hygiene industry has been investing in manufacturing equipment designed to run plastic wrapper materials while achieving high throughput, accuracy, efficiency, and low cost production, using highly optimized materials and processes. On the contrary, the paper used in the manufacture of conventional paper sacks, for example, is processed on equipment designed specifically for converting paper and is converted at speeds that are considerably slower than the speeds used in the production of absorbent hygiene products (converting speeds for absorbent hygiene products may exceed 100 meters/min). Also, conventional Kraft papers (used in, for example, packaging or label release liners) are not sufficiently stretchable to survive the wrapper folding, transporting, and sealing operations that are typically used in the production of absorbent hygiene products without wrinkling, tearing, or having web slack, which results in productivity losses, increased scrap, and generally low quality wrapping.

Furthermore, most existing folding processes involve some stretching of the wrapper web. Generally, to fold the wrapper a folding surface (such as fingers, bars, rollers, idlers, folding boards etc.) may be used to apply a force to the wrapper and create one or more fold lines. It is to be appreciated that any folding surface may be replaced by one or more elements, objects, or media, used to apply forces to create a similar or same folding of the web. Some existing folding processes rely on equal folding path lengths between a first edge of a wrapper web and a second edge of the wrapper web, which runs over an idler to force a folding line. This process may be used to make two or more folds in the wrapper, where the number of folds is generally related to the desired height of the wrapped pad. Alternatively, the folding process may utilize one or more folding boards to fold the wrapper web and the pad. As noted above, most existing folding processes involve some stretching of the wrapper web, and conventional Kraft papers may be prone to tearing in these processes due to their lack of stretch.

Conventional Kraft paper is characterized by a stretch of less than 5%, where stretch is measured as the percent strain of the paper at its breaking point, stretch is calculated as the MD stretch at break. As previously discussed, paper may be creped to add stretch or paper may be selected that has an ability to stretch based on its material properties. With regard to creped paper, the increase in stretch is believed to be due to the microfolds in the creped paper, which may stretch out under tension. The paper wrapper according to the present disclosure preferably comprises a paper, such as a creped paper, having a stretch of at least 9%, preferably at least 10% or greater, or at least 17% or greater, in order to withstand the existing folding processes.

Some folding processes, such as those that utilize equal fold path lengths and idlers to force folding lines, may require a wrapper web to have a folding angle of at least about 45°, or from about 45° to about 90°, or preferably from about 45° to about 65°. The folding angle is related to the bending stiffness of the wrapper web in the folding direction and the caliper of the wrapper web by the following equation: $Folding angle = Arctangent \left(\left(\begin{matrix}2 * 9.81 N / Kg * density * folding \\ {width}^{∧} 3\end{matrix}\right)/\left(bending {modulus * thickness}^{∧}2\right)\right)$ where the density of the wrapper web is calculated by measuring the basis weight of the paper in Kilogram per square meter, following the ISO 536 test method described herein, and dividing the measured basis weight by the measured paper thickness in meters, which is measured using a Thwing-Albert ProGauge Instrument, with a 50.8mm diameter foot and 2.00kPa pressure, and where the bending modulus is calculated as (0.3125*slope)/(compressed caliper^3) and expressed in MegaPascals, where compressed caliper measurements are obtained using a TMI 49-70 high force caliper with a 16mm anvil and 50.40kPa pressure and the slope is obtained using the Ultra Sensitive 3 point Bending Method, described below. The folding width is the distance between the folding line and the edge of the paper expressed in meters. Generally, a material having a high bending stiffness and a high compressed caliper will be characterized by a lower folding angle, which makes the material more difficult to fold, particularly in a folding process that utilizes equal folding path lengths and idlers to force folding lines.

### Coatings

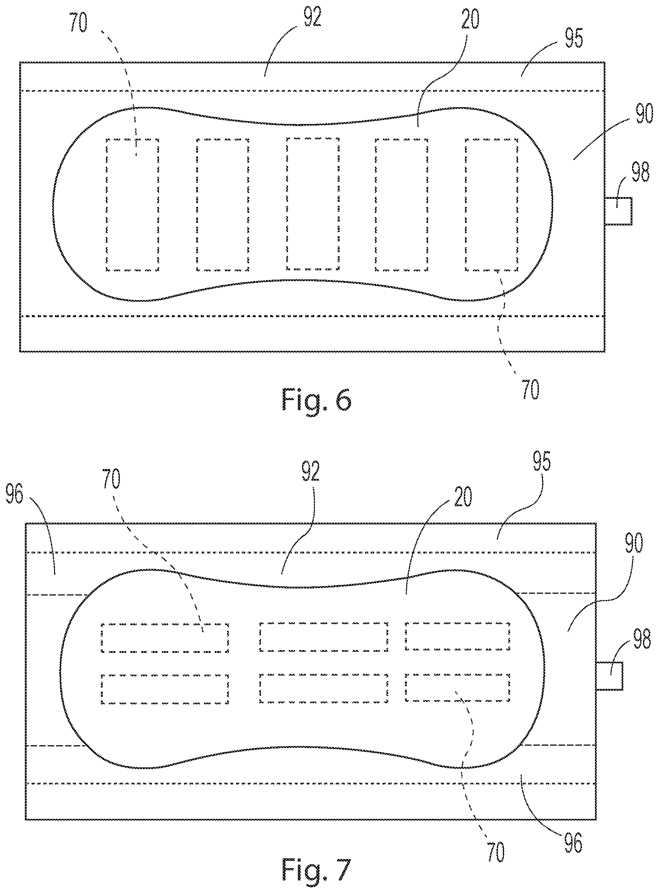

As discussed above, the wrapper of the present disclosure functions to protect the absorbent article and, more specifically, in some embodiments, the adhesive 70 on the outward-facing surface of the backsheet 40 of the absorbent pad 20, as shown in Fig. 2. As such, the inner surface 92 (surface facing the feminine hygiene pad) of the wrapper and/or wrapper web may be coated with a release agent 90, providing a releasably attached protective wrapper for the pad to protect the adhesive prior to use, as shown in Figure 6. Such a protective wrapper also inhibits the adhesive 70 from inadvertently sticking to other portions of the pad 20 prior to use.

The release agent 90 may be applied to the wrapper at an amount of about 0.5 g/m² to about 10 g/ m², or from about 1.0 g/ m² to about 6 g/m², or from about 1.0 g/m² to about 4 g/m², or from about 2 g/m² to about 4 g/m². The amount of release agent 90 will depend on the type, concentration, and effectiveness of the compound aiding the release in the release agent coating formula. As shown in Figure 6, the release agent 90 is applied to at least a portion or a region of the wrapper's inner surface 92 (rather than the entire inner surface of the wrapper), namely the region of the wrapper's inner surface that contacts the adhesive 70 on the pad. The release agent 90 may be applied to the inner surface 92 of the wrapper to the region that contacts the adhesive 70 on the pad and/or a region beyond the region that contacts the adhesive 70. The release agent 90 may be applied to the inner surface 92 of the wrapper such that the release agent does not extend beyond the outer perimeter of the absorbent pad. The release agent 90 may be applied to the inner surface 92 of the wrapper such that the release agent extends beyond at least a portion of the outer perimeter of the absorbent pad.

The wrapper also protects the absorbent pad by sealing the absorbent pad within the wrapper. In order to seal the wrapper of the present disclosure, e.g., paper wrapper, using existing equipment designed to convert plastic film wrapper (the sealing equipment widely used today relies on heat and pressure to bond the plastic film), the wrapper may be coated with a sealing aid. The sealing aid 95 is applied to a portion or a region of the wrapper's surface that need to be sealed to another portion or region of the wrapper's surface. For example, as illustrated in Figure 4, the sealing aid is applied to a portion or a region of the wrapper's inner surface, namely the longitudinal side edges of the wrapper. The sealing aid 95 may be incompatible with the release agent 90, which is also applied to the inner surface of the wrapper, as shown in Fig. 6 (as sealed, shown in Fig. 1). As such, the region of the wrapper's inner surface that contacts the adhesive 70 on the pad (and, optionally, beyond this region), where release agent 90 is disposed, is preferably substantially free of sealing aid 95. Similarly, the inner surfaces of the longitudinal side edges of the wrapper that have sealing aid 95 disposed thereon are preferably substantially free of release agent 90. The sealing aid 95 may substantially abut the release agent 90, as illustrated in Figure 4, or, alternatively, there may be one or more gaps 96 between the sealing aid 95 and the release agent 90, as illustrated in Figure 7.

Sealing aids include cold- and heat-seal adhesives. Heat-seal adhesives are preferred. Sealing aids also include water-based adhesives containing acrylic copolymers, vinyl acetate polymers, or blends thereof with low density polyethylene or high density polyethylene. Preferred sealing aids, such as heat seal adhesives, are also water-soluble to avoid contamination in recycling streams. The preferred sealing aids, such as heat seal adhesives, also have viscosities between 30s and 60s, measured using ASTM D1084 with Zahn EZ #3 cup, where a sample is taken from the reservoir as the adhesive is coated onto the paper. Sealing aids with higher viscosities may contribute to uneven application, inconsistent wrapper opening forces, and difficult and/or noisy opening of the wrapper.

Sealing aids generally do not adhere to surfaces coated with a release agent. Therefore, as discussed above, the release agent is preferably applied to a targeted region of the wrapper's inner surface, namely the region of the wrapper's inner surface that contacts the adhesive 70 on the pad and, optionally, slightly beyond this region. Preferably, the inner surfaces of the longitudinal side edges of the wrapper, wherein the sealing aid is applied, are substantially free of release agent. Importantly, targeting a release agent, particularly a curable silicone release agent, to a region of the wrapper requires specialized application and curing equipment.

Surprisingly, the wrapper according to the present disclosure may utilize a release agent that can be applied using readily available and installable printing equipment, such as flexographic and gravure printing presses. More specifically, the preferred release agents for use with the wrappers according to the present disclosure are substantially free of or free of silicone acrylates, cationic curable silicones, and/or polydimethylsiloxanes, which require nitrogen inerting equipment, ultraviolet lights, or other specialty equipment and may contaminate recycling streams. The release agent may also be substantially free of or free of fluoropolymers. The release agent may comprise an aliphatic modified polyurethane dispersion, a hydrophobic acrylic polymer, a vinyl acrylic copolymer, or a mixture thereof. Bio-based acrylic polymers and other biomass-derived polymers, made from renewable feedstocks like vegetable oils and cellulose, may also be used in the formulation of the release agent.

The release agent may contain natural substances that can provide release by mechanism of electrochemical interactions. Some of these substances are used in the food industry as release agents and may be derived from lecithin, vegetable oil, or animal fat. The release agent may also contain particles, such as clays, silica, and/or ceramics (e.g., hexagonal boron nitride or zinc stearate) to further improve release by increasing the rugosity of the applied-to surface, by dipole electrochemical interactions, or by molecular planar slip. Preferably, the release agent is a water-based and water-soluble formula, which can be easily removed in available paper recycling streams.

Furthermore, paper tends to be porous, and paper that has undergone creping can be even more porous. Thus, coating that are applied to the paper may be absorbed through the pores of the paper, which may reduce the efficacy of the coating or require a greater amount of the coating to be applied. To increase the efficacy of the coating and to minimize the amount of coating, a primer may be applied to the surface of the paper to reduce the porosity of the paper by reducing the size of the pores in the paper. Primers may also improve the appearance of printed graphics, maintaining the definition of lines and reducing dot gains. The preferred primers are water-based and/or water soluble. The primer may be formulated with polymer resins, such as nitrocellulose, polyamides, acrylic polymers, polyurethanes, and adhesion promoters, such as polyimides, silanes, and aziridine. The primer formulation may alco comprise biobased polymers, such as cellulose, starch, chitin, chitosan, xylan, other types of hemicelluloses, and polyesters derived from vegetable oils. The primer formulation may also contain minerals, such as clays, silicas, talc, and calcium carbonate (sometimes referred to as inorganic fillers). The use of primers may be particularly useful for creped paper due to the relatively greater porosity of the paper having been creped. The primer may be used to reduce the porosity of the creped paper by reducing the size of the pores in the paper.

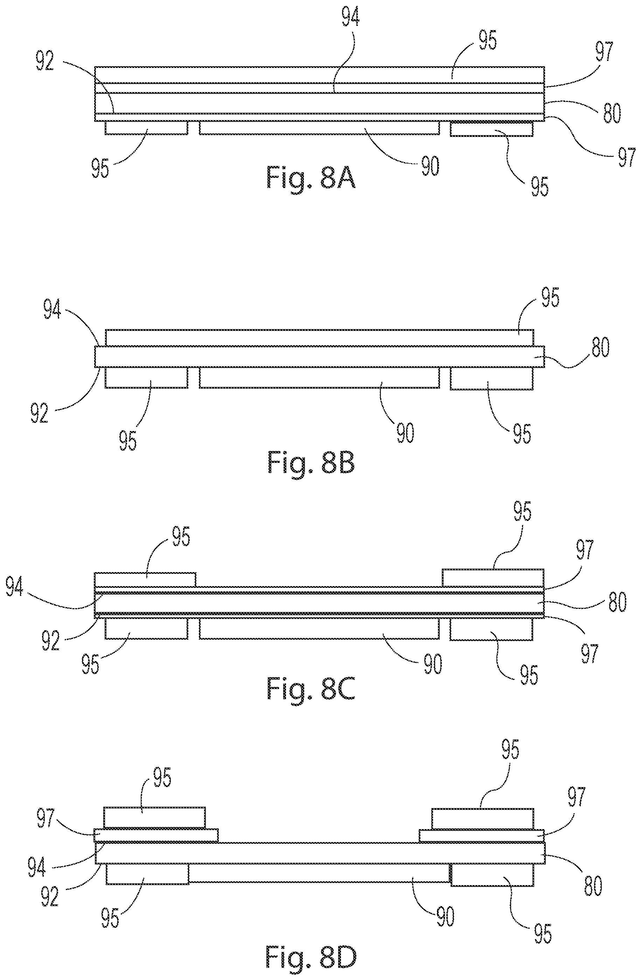

The coatings as discussed herein may be applied to the paper wrapper in various configurations depending on, at least in part, the type of paper, configuration of the fold, and areas of the wrapper to be sealed. In some embodiments, such as illustrated in the cross sectional view of the wrapper 80 in Figure 8A, the wrapper 80, such as a paper wrapper, includes an inner surface 92 and an outer surface 94, such as previously discussed. The inner surface 92 of the wrapper 80 may include a sealing aid 95 disposed in a first portion and a sealing aid 95 disposed in a second portion. The first portion and the second portion including the sealing aid 95 may be adjacent to the outer most longitudinal edges of the wrapper 80. Additionally, a release agent 90 may be disposed on a third portion of the inner surface 92 of the wrapper 80. The release agent 90 may be disposed between the first portion and the second portion including the sealing aid 95. As previously discussed herein, the sealing aid may be disposed on areas that will be used to seal the wrapper when in a folded configuration and the release agent 90 may be used to protect the adhesive on the absorbent pad prior to use and allow for the absorbent pad to be easily and readily removed from the wrapper for use. Additionally or optionally, a primer 97 may be disposed on the inner surface 92 of the wrapper 80. The primer 97 may be applied on the entire inner surface 92 of the wrapper such that the primer spans from adjacent the longitudinal edges and lateral edges of the wrapper. The primer 97 may also be disposed on certain areas of the inner surface 92 of the wrapper such that the primer is not over the entire inner surface 92. For example, the primer may be applied to those areas of the inner surface 92 where the porosity of the wrapper needs to be reduced so that other coatings or materials can be applied to the wrapper without being adversely affected by the pores of the wrapper. For example, the primer 97 may be applied in the first portion and the second portion in substantially the same area as the sealing aid 95, and/or the primer 97 may be applied in substantially the same area as the release agent. Thus, there may be one or more gaps between areas or regions of the primer.

Still referring to Figure 8A, one or more coatings may be disposed on or applied to the outer surface 92 of the wrapper 80. A primer 97 may be applied to the entire outer surface or one or more regions of the outer surface. Additionally, a sealing aid 95 may be applied or disposed on the primer 97. Each of the sealing aid 95 and the primer may be applied or disposed on the entire outer surface or portions of the outer surface. The sealing aid 95 and primer 97 may be applied to the same regions of the outer surface, different regions, or overlapping regions. For example, the primer 97 may be applied on the entire outer surface and the sealing aid may be applied on one or more regions of the outer surface.

Figures 8B-8D illustrate various configurations for the application of coatings. As illustrated in Figure 8B, the wrapper 80 may not include a primer, and the sealing aid 95 and release agent 90 may be applied or disposed directly on the surface of the wrapper 80. Further, it is to be appreciated that the coating may be applied or disposed on the surface of wrapper 80 such that the coating is applied along the longitudinal edge of the wrapper or the coating may be applied such that there is a gap between the coating and the longitudinal edge of the wrapper. As illustrated in Figures 8C and 8D, the coating, such as the sealing aid 95, may be applied or disposed on only a certain region of the wrapper 80. Further, certain coatings are applied to only a certain side of the wrapper 80. For example, the release agent 90 may be applied to only the inner surface 92 of the wrapper 80.

It is also to be appreciated that ink may be applied to the wrapper to form one or more decorative patterns or elements. The ink may be disposed directly on the inner or outer surface of the wrapper. The ink may be disposed on the primer and/or the sealing aid and/or the release coating. In some embodiments, the ink may be disposed on the primer so that the pores of the wrapper become smaller and less ink needs to be applied to get the same visual intensity because the ink does not get absorbed into the pores of the wrapper.

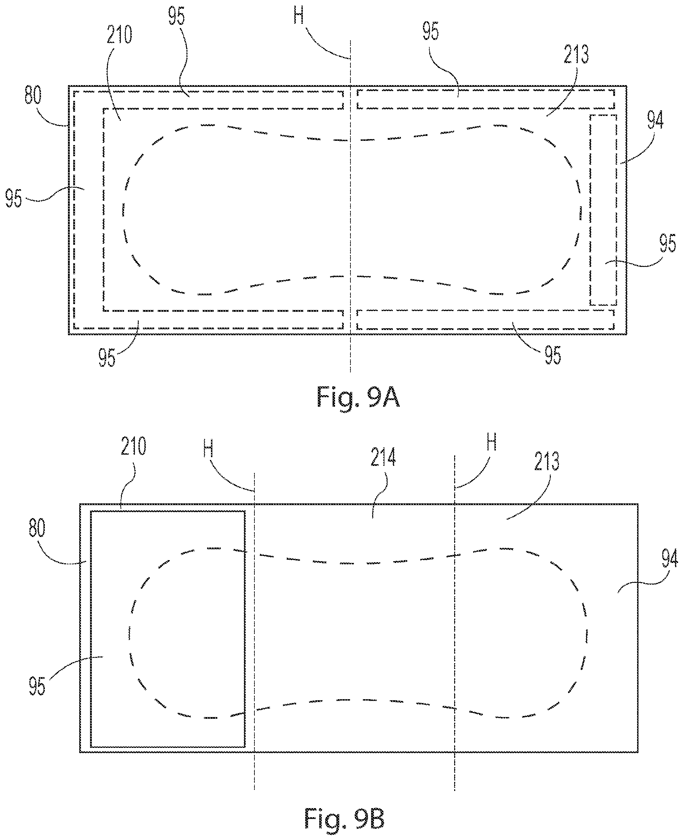

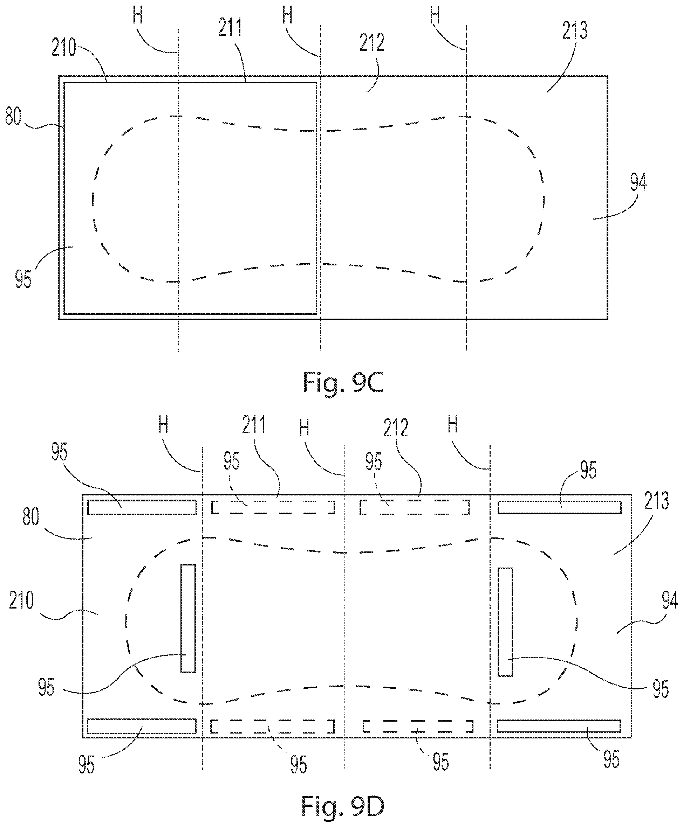

As previously discussed, the placement of the sealing aid 95 may be in regions of the wrapper 80 such that when the wrapper is in a folded configuration, the sealing aid 95 is placed in regions of the wrapper that will be sealed together. Those portions of the wrapper that are sealed together may be sealed by one or more techniques such as ultrasonic bonding, pressure bonding, crimping, and/or thermal bonding. As illustrated in Figure 9A, the wrapper 80 may be folded along a single fold line H. The sealing aid 95 may be disposed on the inner surface 92 of the wrapper 80, in a continuous manner along a portion of the perimeter of the wrapper 80. The portion of the wrapper 80 that includes the sealing aid 95 is the portion that will be sealed. It is to be appreciated that the sealing aid 95 may also be disposed or may alternatively be disposed on the rear section 213. As illustrated in Figure 9B, the wrapper 80 may be folded along a first fold line H and a second fold line H forming a front section 210, a mid section 214, and a rear section 213. The sealing aid 95 may be disposed on the inner surface 92 or the outer surface 94 of the front section 210 such that when the wrapper is folded the front section 210 engages that rear section 213 and a portion of the wrapper in the front section 210 is sealed to the rear section 213. It is also to be appreciated that the sealing aid 95 may also be disposed on the rear section 213 or alternatively be disposed on the mid section 214. The placement of the sealing aid 95 depends on how the wrapper is folded. As illustrated in Figure 9C, the wrapper 80 may be folded along a first fold line H, a second fold line H, and a third fold line H. The sealing aid 95 may be disposed on the front section 210 and the front-mid section 211, as illustrated in Figure 9C. It is also to be appreciated that the sealing aid 95 may be disposed on the rear-mid section 212 and the rear section 213, and the sealing aid may be disposed on the at least one of the inner surface 92 and the outer surface 94. Further, as illustrated in Figure 9D, the sealing aid 95 may be disposed on various portions of the wrapper 80 on the inner surface 92 and/or the outer surface 94. The sealing aid 95 may be discontinuously applied to the wrapper 80 forming discrete regions of the sealing aid 95. The placement of the sealing aid 95 will depend on the type of fold and the regions of the wrapper that are intended to be sealed. The sealing aid 95 allows for the cellulosic wrapper, such as a paper wrapper, to be sealed.

The wrapper of an absorbent pad is preferably easy to open and convenient to use, and the seal of the wrapper is preferably strong enough to survive transport, e.g., inside a purse or a bag. Preferably, the sealing aid creates bonds that open with a force of greater than about 0.5 N and less than about 3.0 N, preferably a force of about 1.0 N to about 2.0 N. The strength of the bonds is measured using a testing machine conforming to ASTM D76-93 standard for constant rate of extension and follow ASTM D1876-08 using a test speed of 300 mm/min and peeling the bond open in the direction perpendicular to the seal in a way that the test length will be equal to the width of the seal. The values expressed above represent the Peak Force recorded over the test length.

For quiet and easy release of the pad from the wrapper, the release peel force of the pad from the inner surface of the wrapper (coated with the release agent) is less than about 5.0 N, or from about 0.5 N to about 3.0 N, preferably less than 3.0 N, and greater than about 1.0 N. The release peel force is measured using a test machine conforming to ASTM D76-93 standard for constant rate of extension, following ASTM D1876-08, loading the pad with the wrapper into the instrument by clamping the wrapper to the movable jaw and the top of the pad to the stationary jaw. The wrapper is peeled from the pad along the long direction of the pad using a test speed of 3000 mm/min.

The wrappers of the present disclosure may be recyclable, to minimize material sent to landfills. There are two known industry standards for the recyclability of paper: The Western Michigan University Recyclability Standard, which is available through the WMU Paper Pilot Plants located in 4651 Campus Dr Kalamazoo MI 49008-5441 USA; and the Papiertechnische Stiftung Test Method PTS-RH 021:2012, which is available thru PTS at Pirnaer Strasse 37, 01809 Heidenau, Germany. As used herein, the term "recyclable" refers to a material that meets a 75% yield of good pulp following the West Michigan University Standard.

### Examples

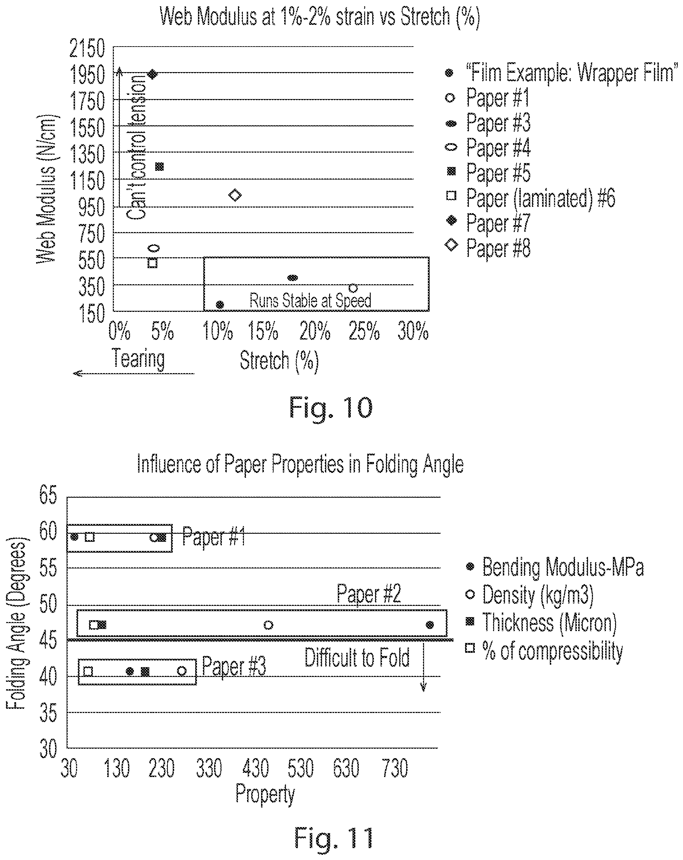

The data in Table 1 below is illustrated graphically in Figure 10. The data in Table 1/Figure 10 shows that a wrapping material having a stretch less than 9% exhibits breakouts and tearing, while a material having a MD Web Modulus greater than 550 N/cm (at 1-2% Strain) exhibits slack and challenges in maintaining web tension.

**Table 1.**

| Sample | Description of Sample | Run Observation | MD Web Modulus (N/cm) at 1-2% Strain | Stretch (%) |
|---|---|---|---|---|
| Film Wrapper | 20 micron, 15 gsm film wrapper (from Always^{®} Ultra Thin, size 1 pad) | Runs at speed, good web tension and stability. | 199.67 | 11% |
| Film Pouch | 38 micron film, available as C669T-300 1.50 Mil white cast pouch film, from Printpack, sold for use in packaging bags and pouches. | Runs at speed, good web tension and stability. | 228.48 | 314.46% |
| Paper #1 | 50gsm, 231 micron, creped paper that is embossed with a diamond pattern, supplied by Billerud Korsnas Beetham Mills, as Steriwrap 2005 (sold as a medical sterilization paper). | Runs at speed, good web tension and stability. | 324.54 | 24% |
| Paper #2 | 47gsm, 102 micron, creped paper, creped by Peltamed and sold as Medicrepe Non-Embossed Lot# 5021811 (Billerud Korsnas). | Runs at speed, good web tension and stability. | 309.98 | 19.50% |
| Paper #3 | 54gsm, 196 micron, creped and wax-coated paper (Non-Embossed) (sold as Peltamed Medicrepe from BillerudKorsnas AB). | Runs at Speed, good web tension and stability | 400.55 | 18% |
| Paper #4 | 29gsm bleached Kraft Paper, available from Liner Brasil Industria E Comercia Ltda and sold for the production of siliconized paper. | Web tearing and breakouts observed, not able to run. | 628.9 | 4% |
| Paper #5 | 32gsm, 80 micron, siliconized bleached Kraft paper, available from Mondi Heerlen B. V. | Web tearing observed, not able to run. | 1245 | 4.7% |
| Paper #6 | 30 MDP laminated paper (laminated with 15 micron caliper polyvinyl acetate polymer coating), available from Nippon Paper Papylia Co. | Web tearing and breakouts observed, not able to run at speed. | 516.82 | 3.93% |
| Paper #7 | 60 gsm bleached Kraft paper, available from Sappi Europe. | Web tearing and breakouts observed, not able to run at speed. | 1936 | 3.96% |
| Paper #8 | 45gsm Peltamed Medicrepe creped paper (Non-Embossed), available from BillerudKorsnas AB) | Web has slack and cannot maintain tension. | 1034.20 | 12% |

The data in Table 2 below is illustrated graphically in Figure 11. The data in Table 2/Figure 11 shows that certain properties of wrapping materials (three sample paper materials described in Table 1) influence the Folding Angle of the material.

**Table 2.**

| Sample | Folding Angle | Compressed Caliper (mm) | Thickness (mm) | Density (Kg/m3) | Bending Modulus-CD (Mpa) | Paper's % of Compressibility (Compressed Caliper/ Thickness) | Folding width (m) |
|---|---|---|---|---|---|---|---|
| Paper #1 | 59.5 | 0.180 | 0.231 | 216.45 | 46.89 | 78 | 0.1 |
| Paper #2 | 47.07 | 0.090 | 0.102 | 460.78 | 808.47 | 88 | 0.1 |
| Paper #3 | 40.54 | 0.145 | 0.196 | 275.51 | 164.52 | 74 | 0.1 |

### Test Methods

### ISO 536 - Basis Weight

The basis weight of a test sample is the mass (in grams) per unit area (in square meters) of a single layer of material and is measured in accordance with compendial method ISO 536. The mass of the test sample is cut to a known area, and the mass of the sample is determined using an analytical balance accurate to 0.0001 grams. All measurements are performed in a laboratory maintained at 23° C.±2 C.° and 50%±2% relative humidity and test samples are conditioned in this environment for at least 2 hours prior to testing.

Measurements are made on test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample must be as large as possible so that any inherent material variability is accounted for.

Measure the dimensions of the single layer test sample using a calibrated steel metal ruler traceable to NIST, or equivalent. Calculate the Area of the test sample and record to the nearest 0.0001 square meter. Use an analytical balance to obtain the Mass of the test sample and record to the nearest 0.0001 gram. Calculate Basis Weight by dividing Mass (in grams) by Area (in square meters) and record to the nearest 0.01 grams per square meter (gsm). In like fashion, repeat for a total of ten replicate test samples. Calculate the arithmetic mean for Basis Weight and report to the nearest 0.01 grams/square meter.

### ISO 1924-3 -Tensile Properties (Tensile Strength, Stretch, Energy Absorption)

The tensile properties (tensile strength, stretch, and energy absorption) of a test sample are calculated from measured force and elongation values obtained using a constant rate of elongation until the sample breaks. The test is run in accordance with compendial method ISO 1924-3, with modifications noted herein. Measurements are made on a constant rate of extension tensile tester using a load cell for which the forces measured are within 1% to 99% of the limit of the cell. A suitable instrument is the MTS Alliance using Test Suite Software, available from MTS Systems Corp., Eden Prairie, Minn., or equivalent. All measurements are performed in a laboratory maintained at 23° C.±2 C.° and 50%±2% relative humidity and test samples are conditioned in this environment for at least 2 hours prior to testing.

Measurements are made on both MD (machine direction) and CD (cross direction) test samples taken from rolls or sheets of the raw material, or test samples obtained from a finished package. When excising the test sample from a finished package, use care to not impart any contamination or distortion to the sample during the process. The excised sample should be free from residual adhesive and taken from an area of the package that is free from any seams or folds. The test sample is cut to a width of 25.4 mm with a length that can accommodate a test span of 50.8 mm. The long side of the sample is parallel to the direction of interest (MD, CD). Normally in finished packages, the MD runs from the bottom to the top of the package, but this can be verified by determining the fiber orientation if in doubt. Ten replicate test samples should be prepared from the MD and ten additional replicates from the CD.

Program the tensile tester for a constant rate of extension uniaxial elongation to break as follows. Set the gauge length (test span) to 50.8 mm using a calibrated gauge block and zero the crosshead. Insert the test sample into the grips such that the long side is centered and parallel to the central pull axis of the tensile tester. Raise the crosshead at a rate of 508 mm/min until the test sample breaks, collecting force (N) and extension (mm) data at 100 Hz throughout the test. Construct a graph of force (N) versus extension (mm).

Read the maximum force (N) from the graph and record as Peak Force to the nearest 0.1 N, noting MD or CD. Read the extension at the maximum force (N) from the graph and record as Elongation at Break to the nearest 0.01 mm, noting MD or CD.

Calculate the arithmetic mean Peak Force for all MD replicates and then all CD replicates and record respectively as Mean MD Peak Force and Mean CD Peak Force to the nearest 0.1 N. Calculate the arithmetic mean Elongation at Break for all MD replicates and then all CD replicates and record respectively as Mean MD Elongation at Break and Mean CD Elongation at Break to the nearest 0.01 mm.

Tensile strength is calculated by dividing the Mean Peak Force (N) by the width of the test sample (25.4 mm). Calculate the tensile strength for the MD replicates and then the CD replicates and report respectively as MD Tensile Strength and CD Tensile Strength to the nearest 0.1 kN/m.

Stretch at break is calculated by dividing the Mean Elongation at Break (mm) by the initial test length (test span) of 50.8 mm, and then multiplying by 100. Calculate the stretch at break for the MD replicates and then the CD replicates and report respectively as MD Stretch at Break and CD Stretch at Break to the nearest percent.

Web Modulus in MD: Take the force data (N) and divide by the width of the specimen, which in this case is 2.54 cm to obtain Force per cm. Take the extension data (mm), divide by the initial test length of 50.8mm to obtain the strain (mm/mm). Construct a graph of Force per mm by strain. Calculate the slope of the curve between the 1%-2%, 2%-3%, 3%-4% and 4%-5% strain intervals and report the results in N/cm.

### Ultra Sensitive 3 Point Bending Method

The bending properties of a test sample are measured using an Ultra Sensitive 3 Point Bend Test on a universal constant rate of extension test frame (a suitable instrument is the MTS Alliance using TestSuite Software, as available from MTS Systems Corp., Eden Prairie, MN, or equivalent) equipped with a load cell appropriate for the forces being measured. The test is executed on test specimens prepared for both MD (machine direction; parallel to the longitudinal axis of the absorbent article) and CD (cross direction; perpendicular to the longitudinal axis of the absorbent article) bending. All testing is performed in a room controlled at 23°C ± 3°C and 50% ± 2% relative humidity.

The ultra-sensitive 3 point bend method is designed to maximize the force signal to noise ratio when testing materials with very low bending forces. The force signal is maximized by using a high sensitivity load cell (e.g., 5N), using a small span (load is proportional to the span cubed) and using a wide specimen width (total measured load is directly proportional to width). The fixture is designed such that the bending measurement is performed in tension, allowing the fixture mass to be kept to a minimum. Noise in the force signal is minimized by holding the load cell stationary to reduce mechanical vibration and inertial effect and by making the mass of the fixture attached to the load cell as low as possible.

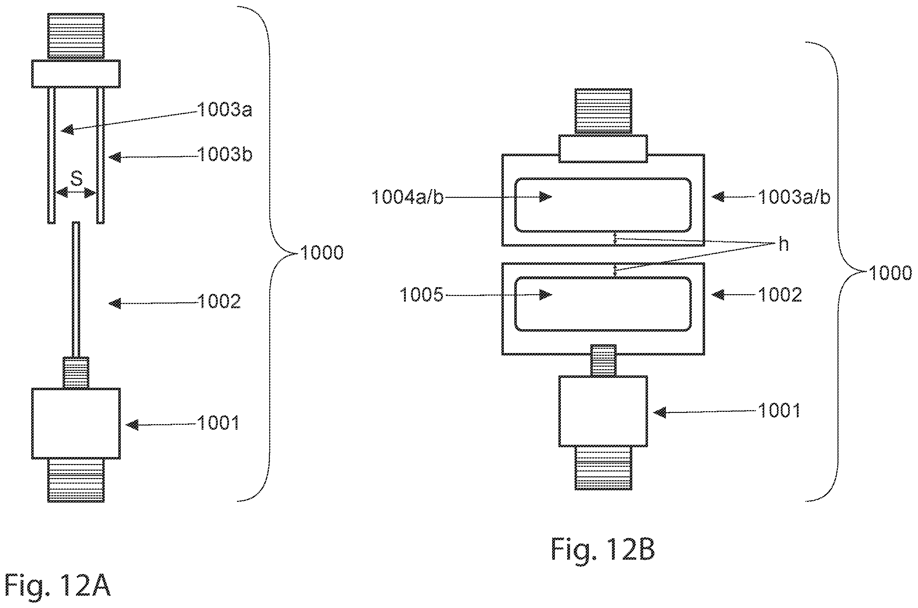

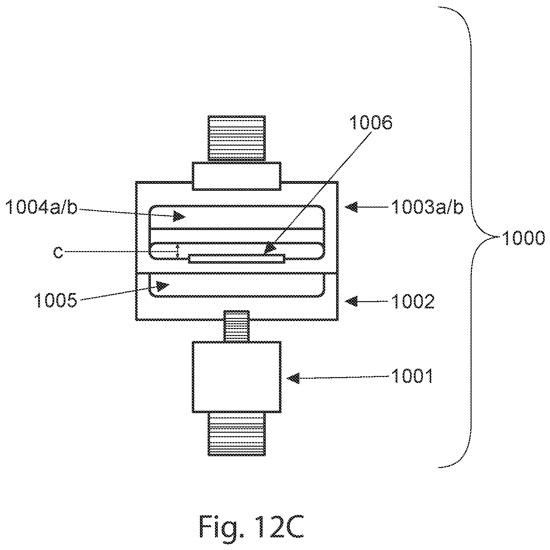

Referring to Figures 12A-12C, the load cell 1001 is mounted on the stationary crosshead of the universal test frame. The ultra-sensitive fixture 1000 consists of three thin blades constructed the inner horizontal edges of cavities 1004a and 1004b in the outside blades 1003a and 1003b and the inner horizontal edge of cavity 1005 in the central blade 1002 (see Figure 7c). The specimen 1006 is placed within clearance c such that it spans the inner horizontal edges of cavities 1004a and 1004b in the outside blades 1003a and 1003b. For MD bending, the test specimen is oriented such that the CD (short side) of the specimen is perpendicular to the horizontal edges of the blades, and the article-facing surface of the specimen is facing up. For CD bending, the test specimen is oriented such that the MD (short side) of the specimen is perpendicular to the horizontal edges of the blades and the article-facing surface of the specimen is facing up. Center the specimen 1006 between the outside blades 1003a and 1003b. Slowly move the outside blades 1003a and 1003b in a direction opposite of the stationary crosshead until the inner horizontal edge of cavity 1005 in the central blade 1002 touches the top surface of the specimen 1006. Start the test and continuously collect force and displacement data.

Force (N) is plotted versus displacement (mm). The maximum peak force is recorded to the nearest 0.001 N. The slope of the linear portion of the force versus displacement curve is determined and recorded as slope to the nearest 0.001 N/mm. The area under the curve from load onset up to the maximum peak force is calculated and recorded as energy to peak to the nearest 0.001 N-mm. In like fashion, repeat the entire test sequence for a total of five MD bending test specimens and five CD bending test specimens.

For each test specimen type (MD and CD), the arithmetic mean of the maximum peak force among like specimens is calculated to the nearest 0.001 N and recorded as MD Peak Load and CD Peak load, respectively. For each test specimen type (MD and CD), the arithmetic mean of the slope among like specimens is calculated to the nearest 0.001 N/mm and reported as MD Slope and CD Slope, respectively. For each test specimen type (MD and CD), the arithmetic mean of the energy to peak among like specimens is calculated to the nearest 0.001 N-mm and reported as MD Energy to Peak and CD Energy to Peak, respectively.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An individually packaged absorbent article comprising:
an absorbent article (20) comprising a liquid permeable topsheet (30), a liquid impermeable backsheet (40), and an absorbent layer (50) disposed between the topsheet (30) and the backsheet (40), wherein the outward-facing surface of the backsheet (40) has an adhesive (70) disposed thereon; and
a wrapper (80) overlaying the outward-facing surface of the backsheet (40) and releasably affixed to the adhesive (70) disposed thereon, wherein the wrapper (80) comprises a sheet material having a basis weight of about 30 gsm to about 85 gsm according to the ISO 536 - Basis Weight Method described herein, wherein the sheet material comprises about 70% to about 100% natural fibers and has a folding angle of about 45° to about 90° according to the following formula: 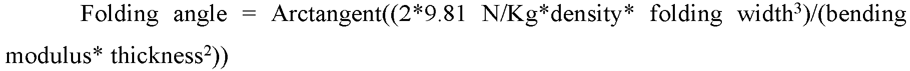 Folding angle = Arctangent((2*9.81 N/Kg*density* folding width3)/(bending modulus* thickness2))
wherein the density is calculated by measuring the basis weight of the paper in Kilogram per square meter, following the ISO 536 - Basis Weight Method described herein, and dividing the measured basis weight by the measured paper thickness in meters, which is measured using a Thwing-Albert ProGauge Instrument, with a 50.8mm diameter foot and 2.00kPa pressure,
wherein the bending modulus is calculated as (0.3125*slope)/(compressed caliper³) and expressed in MegaPascals, where compressed caliper measurements are obtained using a TMI 49-70 high force caliper with a 16mm anvil and 50.40kPa pressure and the slope is obtained using the Ultra Sensitive 3 point Bending Method described herein, and
wherein folding width is the distance between the folding line and the edge of the paper expressed in meters,
wherein the individually packaged absorbent article (20) is provided in a folded configuration, the absorbent article (20) and the wrapper (80) folded together about at least one fold line;
wherein the sheet material comprises creped paper;
wherein the creped paper has a MD web modulus at 1%-2% strain of less than 450 N/cm and a stretch of at least 9% according to the Ultra Sensitive 3 point Bending Method described herein.

2. The packaged absorbent article according to claim 1, wherein the folding angle is from about 45° to about 65°.

3. The packaged absorbent article according to any one of the preceding claims, wherein a release agent (90) is deposited on the inner surface (92) of the wrapper (80) in a region that contacts the adhesive (70) disposed on the backsheet (40) of the absorbent article (20).

4. The packaged absorbent article according to claim 3, wherein the release peel force between the inner surface (92) of the wrapper (80) and the backsheet (40) of the absorbent article (20) including the adhesive is less than about 5.0 N measured using a test machine conforming to ASTM D76-93 standard for constant rate of extension, following ASTM D1876-08, loading the pad with the wrapper into the instrument by clamping the wrapper to the movable jaw and the top of the pad to the stationary jaw, then the wrapper is peeled from the pad along the long direction of the pad using a test speed of 3000 mm/min.

5. The packaged absorbent article according to any one of the preceding claims, wherein a sealing aid (95) is deposited on the inner surfaces (92) of the longitudinal side edges of the wrapper (80).

6. The packaged absorbent article according to claim 5, wherein the sealing aid (95) is bonded such that the wrapper (80) has an opening force of greater than about 0.5 N to about 3.0 N measured using a test machine conforming to ASTM D76-93 standard for constant rate of extension, following ASTM D1876-08 using a test speed of 300 mm/min and peeling the bond open in the direction perpendicular to the seal in a way that the test length will be equal to the width of the seal; wherein values represent the Peak Force recorded over the test length.

7. The packaged absorbent article according to claim 5, wherein the inner surfaces (92) of the longitudinal side edges of the wrapper (80) are substantially free of release agent (90).

8. The packaged absorbent article according to claim 5, wherein the longitudinal side edges of the wrapper (80) are heat and pressure sealed.

9. The packaged absorbent article according to claim 1, wherein the paper comprises about 50% by weight to about 80% by weight of softwood fibers and about 20% by weight to about 50% by weight of hardwood fibers.

10. The packaged absorbent article according to any one of the preceding claims, wherein at least one of a primer (97), which is applied to the surface of the paper to reduce the porosity of the paper by reducing the size of the pores in the paper, a release agent (90), and a sealing aid (95) are disposed on at least one of a front section (210), a mid section (211) between the front section (210) and a rear section (213), and the rear section of the wrapper (80).

11. The packaged absorbent article according to any one of the preceding claims, wherein a primer (97), which is applied to the surface of the paper to reduce the porosity of the paper by reducing the size of the pores in the paper, and a sealing aid (95) is disposed on a front section (210), and wherein the primer (97) is positioned between the sealing aid (95) and a surface of the wrapper (80).

12. The packaged absorbent article according to any one of the preceding claims, wherein a primer (97), which is applied to the surface of the paper to reduce the porosity of the paper by reducing the size of the pores in the paper, and a sealing aid (95) is disposed on a rear section (213), and wherein the primer (97) is positioned between the sealing aid (95) and a surface of the wrapper (80).

## Patentansprüche

1. Einzeln verpackter Absorptionsartikel, umfassend:
einen Absorptionsartikel (20), umfassend eine flüssigkeitsdurchlässige Oberschicht (30), eine flüssigkeitsundurchlässige Unterschicht (40) und eine Absorptionsschicht (50), die zwischen der Oberschicht (30) und der Unterschicht (40) eingerichtet ist, wobei die nach außen weisende Oberfläche der Unterschicht (40) ein darauf eingerichtetes Haftmittel (70) aufweist; und
eine Schutzhülle (80), die über der nach außen weisenden Oberfläche der Unterschicht (40) liegt und an dem darauf eingerichteten Haftmittel (70) lösbar befestigt ist, wobei die Schutzhülle (80) ein Lagenmaterial umfasst, das ein Flächengewicht von etwa 30 g/m² bis etwa 85 g/m² gemäß des hierin beschriebenen ISO 536 - Flächengewichtsverfahrens aufweist, wobei das Lagenmaterial etwa 70 % bis etwa 100 % Naturfasern umfasst und einen Faltungswinkel von etwa 45° bis etwa 90° gemäß der folgenden Formel aufweist: 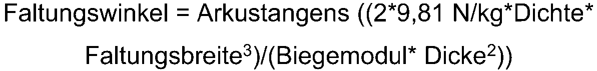 Faltungswinkel = Arkustangens ((2*9,81 N/kg*Dichte* Faltungsbreite3)/(Biegemodul* Dicke2))
wobei die Dichte durch Messen des Flächengewichts des Papiers in Kilogramm pro Quadratmeter, dem hierin beschriebenen ISO 536 - Flächengewichtsverfahren folgend, und durch Dividieren des gemessenen Flächengewichts durch die gemessene Papierdicke in Metern, die unter Verwendung eines Thwing-Albert-ProGauge-Geräts mit einem Fuß von 50,8 mm Durchmesser und 2,00 kPa Druck gemessen wird, berechnet wird,
wobei der Biegemodul als (0,3125*Steigung)/(komprimierte Dicke³) berechnet und in Megapascal ausgedrückt wird, wobei Messungen der komprimierten Dicke unter Verwendung eines TMI 49-70 Hochkraft-Dickenmessgeräts mit einem 16-mm-Amboss und 50,40 kPa Druck erhalten werden und die Steigung unter Verwendung des hierin beschriebenen hochempfindlichen 3-Punkt-Biegeverfahrens erhalten wird, und
wobei die Faltungsbreite der Abstand zwischen der Faltungslinie und der Papierkante, ausgedrückt in Metern, ist,
wobei der einzeln verpackte Absorptionsartikel (20) in einer gefalteten Konfiguration bereitgestellt wird, wobei der Absorptionsartikel (20) und die Schutzhülle (80) zusammen um mindestens eine Faltungslinie gefaltet sind;
wobei das Lagenmaterial gekrepptes Papier umfasst;
wobei das gekreppte Papier einen Maschinenlaufrichtungs-Bahnmodul bei 1 %-2 % Belastung von weniger als 450 N/cm und eine Dehnung von mindestens 9 % gemäß dem hierin beschriebenen hochempfindlichen 3-Punkt-Biegeverfahren aufweist.

2. Verpackter Absorptionsartikel nach Anspruch 1, wobei der Faltungswinkel von ungefähr 45° bis ungefähr 65° beträgt.

3. Verpackter Absorptionsartikel nach einem der vorstehenden Ansprüche, wobei ein Trennmittel (90) auf der Innenoberfläche (92) der Schutzhülle (80) in einem Bereich angelagert ist, der das Haftmittel (70) kontaktiert, der auf der Unterschicht (40) des Absorptionsartikels (20) eingerichtet ist.

4. Verpackter Absorptionsartikel nach Anspruch 3, wobei die Freisetzungsschälkraft zwischen der Innenoberfläche (92) der Schutzhülle (80) und der Unterschicht (40) des Absorptionsartikels (20) einschließlich des Haftmittels weniger als etwa 5,0 N beträgt, gemessen unter Verwendung einer Prüfmaschine, die mit dem ASTM D76-93-Standard für eine konstante Verlängerungsrate, ASTM D1876-08 folgend, konform ist, wobei die Beladung der Einlage mit der Schutzhülle in das Gerät durch Einspannen der Schutzhülle in die bewegliche Backe und des Oberteils der Einlage in die stationäre Backe erfolgt, wobei dann die Schutzhülle von der Einlage entlang der Längsrichtung de Einlage unter Verwendung einer Prüfgeschwindigkeit von 3000 mm/min abgeschält wird.

5. Verpackter Absorptionsartikel nach einem der vorstehenden Ansprüche, wobei ein Versiegelungshilfsmittel (95) auf den Innenoberflächen (92) der Längsseitenränder der Schutzhülle (80) angelagert ist.

6. Verpackter Absorptionsartikel nach Anspruch 5, wobei das Versiegelungshilfsmittel (95) derart gebunden ist, dass die Schutzhülle (80) eine Öffnungskraft von mehr als etwa 0,5 N bis etwa 3,0 N aufweist, gemessen unter Verwendung einer Prüfmaschine, die mit dem ASTM D76-93-Standard für eine konstante Verlängerungsrate, ASTM D1876-08 folgend, konform ist, unter Verwendung einer Prüfgeschwindigkeit von 300 mm/min und Abschälen zum Öffnen der Bindung in der Richtung senkrecht zu der Versiegelung auf eine Weise, dass die Prüflänge gleich der Breite der Versiegelung ist; wobei Werte die über die Prüflänge aufgezeichnete Peak-Kraft darstellen.

7. Verpackter Absorptionsartikel nach Anspruch 5, wobei die Innenoberflächen (92) der Längsseitenränder der Schutzhülle (80) im Wesentlichen frei von Trennmittel (90) sind.

8. Verpackter Absorptionsartikel nach Anspruch 5, wobei die Längsseitenränder der Schutzhülle (80) wärme- und druckverschweißt sind.

9. Verpackter Absorptionsartikel nach Anspruch 1, wobei das Papier zu etwa 50 Gew.-% bis etwa 80 Gew.-% Weichholzfasern und zu etwa 20 Gew.-% bis etwa 50 Gew.-% Hartholzfasern umfasst.

10. Verpackter Absorptionsartikel nach einem der vorstehenden Ansprüche, wobei mindestens eines von einem Primer (97), der auf die Oberfläche des Papiers aufgebracht ist, um die Porosität des Papiers durch Verringern der Größe der Poren in dem Papier zu reduzieren, einem Trennmittel (90) und einem Versiegelungshilfsmittel (95) auf mindestens einem von einem vorderseitigen Abschnitt (210), einem Mittelabschnitt (211) zwischen dem vorderseitigen Abschnitt (210) und einem rückseitigen Abschnitt (213) und dem rückseitigen Abschnitt der Schutzhülle (80) eingerichtet ist.

11. Verpackter Absorptionsartikel nach einem der vorstehenden Ansprüche, wobei ein Primer (97), der auf der Oberfläche des Papiers aufgebracht ist, um die Porosität des Papiers durch Verringern der Größe der Poren in dem Papier zu verringern, und ein Versiegelungshilfsmittel (95) auf einem vorderseitigen Abschnitt (210) eingerichtet sind, und wobei der Primer (97) zwischen dem Versiegelungshilfsmittel (95) und einer Oberfläche der Schutzhülle (80) angeordnet ist.

12. Verpackter saugfähiger Artikel nach einem der vorstehenden Ansprüche, wobei ein Primer (97), der auf der Oberfläche des Papiers aufgebracht ist, um die Porosität des Papiers durch Verringern der Größe der Poren in dem Papier zu verringern, und eine Skalierungshilfsmittel (95) an einem rückseitigen Abschnitt (213) eingerichtet sind, und wobei der Primer (97) zwischen dem Versiegelungshilfsmittel (95) und einer Oberfläche der Schutzhülle (80) angeordnet ist.

## Revendications

1. Article absorbant conditionné individuellement comprenant :
un article absorbant (20) comprenant une feuille de dessus (30) perméable aux liquides, une feuille de fond (40) imperméable aux liquides, et une couche absorbante (50) disposée entre la feuille de dessus (30) et la feuille de fond (40), dans lequel la surface tournée vers l'extérieur de la feuille de fond (40) a un adhésif (70) disposé sur celle-ci ; et
un enveloppeur (80) recouvrant la surface tournée vers l'extérieur de la feuille de fond (40) et fixé de manière amovible à l'adhésif (70) disposé sur celle-ci, dans lequel l'enveloppeur (80) comprend un matériau en feuille ayant un poids de base d'environ 30 g/m² à environ 85 g/m² selon la norme ISO 536 - Procédé de poids de base décrit ici, dans lequel le matériau en feuille comprend environ 70 % à environ 100 % de fibres naturelles et a un angle de pliage d'environ 45 ° à environ 90 ° selon la formule suivante : 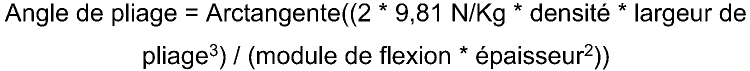 Angle de pliage = Arctangente((2 * 9,81 N/Kg * densité * largeur de pliage3) / (module de flexion * épaisseur2))
dans lequel la masse volumique est calculée en mesurant le grammage du papier en kilogramme par mètre carré, selon la méthode du grammage de base de la norme ISO 536 décrite dans le présent document, et en divisant le grammage mesuré par l'épaisseur de papier mesurée en mètres, qui est mesurée à l'aide d'un instrument ProGauge de Thwing-Albert, avec un pied de 50,8 mm de diamètre et une pression de 2,00 kPa,
dans lequel le module de flexion est calculé en tant que (0,3125 * pente) / (épaisseur comprimée³) et exprimé en mégaPascals, où les mesures d'épaisseur comprimée sont obtenues à l'aide d'une épaisseur à haute force TMI 49-70 avec une enclume de 16 mm et une pression de 50,40 kPa et la pente est obtenue à l'aide de la méthode de flexion ultra-sensible en 3 points décrite ici, et
dans lequel la largeur de pliage est la distance entre la ligne de pliage et le bord du papier, exprimée en mètres,
dans lequel l'article absorbant emballé individuellement (20) est fourni dans une configuration pliée, l'article absorbant (20) et la cape (80) pliés ensemble autour d'au moins une ligne de pliage ;
dans lequel le matériau en feuille comprend du papier crêpe ;
dans lequel le papier crêpé a un module de bande MD à 1 % à 2 % de déformation inférieur à 450 N/cm et un étirement d'au moins 9 % selon le procédé de pliage ultra-sensible en 3 points décrit ici.

2. Article absorbant conditionné selon la revendication 1, dans lequel l'angle de pliage est d'environ 45 ° à environ 65 °.

3. Article absorbant conditionné selon l'une quelconque des revendications précédentes, dans lequel un agent de démoulage (90) est déposé sur la surface interne (92) de l'enveloppeur (80) dans une région qui entre en contact avec l'adhésif (70) disposé sur la feuille arrière (40) de l'article absorbant (20).

4. Article absorbant conditionné selon la revendication 3, dans lequel la force de pelage de libération entre la surface interne (92) de l'enveloppeur (80) et la feuille arrière (40) de l'article absorbant (20) comportant l'adhésif est inférieure à environ 5,0 N mesurée à l'aide d'une machine d'essai conforme à la norme ASTM D76-93 pour un taux d'extension constant, suivant ASTM D1876-08, charger le tampon avec l'enveloppeur dans l'instrument en serrant l'enveloppeur sur la mâchoire mobile et le haut du tampon sur la mâchoire fixe, puis l'enveloppeur est décollé du tampon le long de la direction longue du tampon à l'aide d'une vitesse d'essai de 3000 mm/min.

5. Article absorbant conditionné selon l'une quelconque des revendications précédentes, dans lequel un adjuvant d'étanchéité (95) est déposé sur les surfaces internes (92) des bords latéraux longitudinaux de l'emballage (80).

6. Article absorbant conditionné selon la revendication 5, dans lequel l'aide au scellage (95) est liée de telle sorte que l'enveloppeur (80) a une force d'ouverture supérieure à environ 0,5 N à environ 3,0 N mesuré à l'aide d'une machine d'essai conforme à la norme ASTM D76-93 pour un taux d'extension constant, suivant la norme ASTM D1876-08 à l'aide d'une vitesse d'essai de 300 mm/min et le décollage de la liaison pour l'ouvrir dans la direction perpendiculaire au joint d'étanchéité de manière à ce que la longueur d'essai soit égale à la largeur du joint d'étanchéité ; dans lequel les valeurs représentent la force maximale enregistrée sur la longueur d'essai.

7. Article absorbant conditionné selon la revendication 5, dans lequel les surfaces intérieures (92) des bords latéraux longitudinaux de l'enveloppeur (80) sont sensiblement exemptes d'agent de démoulage (90).

8. Article absorbant conditionné selon la revendication 5, dans lequel les bords latéraux longitudinaux de l'enveloppeur (80) sont scellés à chaud et sous pression.

9. Article absorbant conditionné selon la revendication 1, dans lequel le papier comprend environ 50 % en poids à environ 80 % en poids de fibres de résineux et environ 20 % en poids à environ 50 % en poids de fibres de feuillus.

10. Article absorbant conditionné selon l'une quelconque des revendications précédentes, dans lequel au moins l'un parmi un apprêt (97), qui est appliqué à la surface du papier pour réduire la porosité du papier en réduisant la taille des pores dans le papier, un agent de démoulage (90) et un adjuvant d'étanchéité (95) sont disposés sur au moins l'une parmi une section avant (210), une section médiane (211) entre la section avant (210) et une section arrière (213), et la section arrière de l'enveloppeur (80).

11. Article absorbant conditionné selon l'une quelconque des revendications précédentes, dans lequel un apprêt (97), qui est appliqué à la surface du papier pour réduire la porosité du papier en réduisant la taille des pores dans le papier, et un auxiliaire d'étanchéité (95) est disposé sur une section avant (210), et dans lequel l'apprêt (97) est positionné entre l'auxiliaire d'étanchéité (95) et une surface de l'enveloppeur (80).

12. Article absorbant conditionné selon l'une quelconque des revendications précédentes, dans lequel un apprêt (97), qui est appliqué sur la surface du papier pour réduire la porosité du papier en réduisant la taille des pores dans le papier, et un agent d'entartrage (95) est disposé sur une section arrière (213), et dans lequel l'apprêt (97) est positionné entre l'aide au scellage (95) et une surface de l'enveloppeur (80).
